(19) Europäisches Patentamt
European Patent Office
Office européen des brevets

(11) EP 3 644 933 B1

# (12) EUROPEAN PATENT SPECIFICATION

(45) Date of publication and mention of the grant of the patent:
**27.12.2023 Bulletin 2023/52**

(21) Application number: **18743249.7**

(22) Date of filing: **28.06.2018**

(51) International Patent Classification (IPC):
***A61F 13/511*** (2006.01) ***A61F 13/512*** (2006.01)
***A61F 13/513*** (2006.01) ***A61F 13/15*** (2006.01)
***A61L 15/18*** (2006.01) ***A61L 15/26*** (2006.01)

(52) Cooperative Patent Classification (CPC):
**A61F 13/51113; A61F 13/15203; A61F 13/512; A61F 13/513; A61L 15/18; A61L 15/42**

(86) International application number:
**PCT/US2018/039894**

(87) International publication number:
**WO 2019/006057 (03.01.2019 Gazette 2019/01)**

## (54) DISPOSABLE ABSORBENT ARTICLE HAVING SURFACE MODIFIED TOPSHEET

SAUGFÄHIGER EINWEGARTIKEL MIT OBERFLÄCHENMODIFIZIERTER DECKSCHICHT

ARTICLE ABSORBANT JETABLE AYANT UNE FEUILLE SUPÉRIEURE MODIFIÉE EN SURFACE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2017 US 201762527368 P**

(43) Date of publication of application:
**06.05.2020 Bulletin 2020/19**

(73) Proprietor: **The Procter & Gamble Company**
**Cincinnati, OH 45202 (US)**

(72) Inventors:
- **AVILES, Misael Omar**
  **Cincinnati, Ohio 45202 (US)**
- **GIZAW, Yonas**
  **Cincinnati, Ohio 45202 (US)**
- **WENNING, Genevieve Cagalawan**
  **Cincinnati, Ohio 45202 (US)**
- **HAMMONS, John Lee**
  **Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-2015/066261** **WO-A1-2016/138277**
**US-A1- 2005 154 362** **US-A1- 2012 089 110**
**US-A1- 2013 197 462**

- **Swanscandles: "W-835 Microcrystalline Wax Slab", , 1 January 2015 (2015-01-01), XP055503477, Retrieved from the Internet: URL:http://www.swanscandles.com/store/w-835_microcrystalline_wax_10lb_slab.htm [retrieved on 2018-08-30]**
- **CABOT: "CAB-O-SIL FUMED SILICA IN EPOXY COATINGS", , 22 September 2015 (2015-09-22), XP055503200, Retrieved from the Internet: URL:http://www.cabotcorp.com/~/media/files/guides/fumed-metal-oxides/application-guide-cab-o-sil-fumed-silica-in-epoxy-coating s.pdf**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 644 933 B1

## Description

FIELD

The present invention pertains to disposable absorbent articles suitable for absorbing and containing body exudates.

BACKGROUND

Disposable absorbent articles are a staple in households around the world since they are used by people of all ages. Babies and toddlers wear such articles to absorb urine and bowel movements. Young girls and women wear pantiliners to manage discharge of vaginal discharge on a daily basis. These same girls and women rely on sanitary napkins each month as they experience their menstrual cycle, which entails their bodies expelling menstrual fluid, on periodic basis over the course of a number of days. Adult men and women find themselves in need of incontinence pads or pants as they get older and are unable to control their urge to urinate. Thus, it is clear that no matter what the age, people often times rely on disposable absorbent articles to help them deal with managing the expulsion of bodily fluids. In the case of babies and toddlers, discretion is not necessarily an important factor of a well-performing article during wear since such wearers rely on an adult to change them and the image of the babies or toddlers are not tarnished if they are found to have spoiled diapers. In fact, this is the expectation and it is even celebrated by parents and caregivers. Adult wearers, on the other hand, pride their discretion when wearing such articles and prefer that such articles go unnoticed by others around them and in an ideal situation, the wearers themselves will forget they are wearing such articles.

Despite this difference in the two different populations of wearers, they (and their care givers) share a common desire for a product that feels dry during wear and even more importantly during use. Dryness during wear and use amounts to comfort and can translate to heightened confidence in a wearer that can appreciate the benefit. Oftentimes, the groin area that is contacted and/or covered by the absorbent article during use may experience a bit of warmth and moisture due to the thick and sometimes lower breathability exhibited by such articles. These conditions are only worsened once they undergo insult by bodily fluids. In order to combat this eventuality of fluid management, it is preferred that the absorbent articles quickly absorb the fluids soon after expulsion from the body. Many manufacturers of such products focus on improving the absorbent materials included therein to increase the acquisition speed of the products which can result in the wearer not experiencing a wet feeling post insult. These improvements have been focused on changes to the materials used for the topsheet, secondary topsheet, acquisition layer, or core of the product. Although many such products quickly acquire the fluids which have been expelled, these same products have not yet dealt with addressing the tendency of expelled fluids to readily spread over the body contacting surface of the product. Such spreading also contributes to the wet feeling some experience.

US 2012/089110 is concerned with an absorbent article, having a liquid pervious topsheet, a backsheet, and an absorbent core. The absorbent article has a lotion composition applied to at least a portion of the outer surface of the topsheet, the lotion composition including a rheology structurant selected from the group consisting of microcrystalline wax, alkyl dimethicone, ethylene glycol dibehenate, ethylene glycol distearate, glycerol tribehenate, glycerol tristearate, and ethylene bisoleamide.

WO 2015/066261 is concerned with is concerned with a material having one-way valve properties including a nonwoven substrate having a first surface having a first surface hydrohead value and a second surface having a second surface hydrohead value; and a superhydrophobic formulation disposed on the first surface, wherein the first surface hydrohead value is less than about 1 cm, and wherein the second surface hydrohead value is at least 4 cm greater than the first surface hydrohead value.

In view of the recognition of this additional contributor to the undesirable wet feeling wearers experience post insult, there seems to be a need for an improvement in disposable absorbent articles that results in minimizing the spread of expelled fluids on the body contacting surface.

SUMMARY

Disposable absorbent articles in accordance with the present invention are well suited for providing a minimized skin contact area of expelled fluid during wear. In one embodiment, a disposable absorbent article comprises an apertured nonwoven topsheet having a body surface and an opposing garment surface, wherein said body surface has an effective aperture area of greater than 1.8 $mm^2$ and less than 3.5 $mm^2$; a backsheet; an absorbent core disposed between said topsheet and said backsheet; and a surface modifying composition disposed on said body surface of said topsheet, wherein the composition comprises hydrophobic particles and a hydrophobic binder in a ratio of binder to particles of 4:1 to 1:4 and wherein, post insult, the article has a Topsheet Stain Area of less than 30 $mm^2$.

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which the designations are used to designate substantially identical elements and in which:

Figure 1 illustrates a perspective view of a disposable absorbent article of the present invention, which is a sanitary napkin.
Figure 2 illustrates a cross section view of the sanitary napkin of Figure 1.
Figure 3 depicts a plot of the Stain Area on a removed topsheet of an article of the present invention.
Figure 4 shows a perspective view of a strikethrough plate useful for the determination of Acquisition Time, which is measured in certain embodiments of the present invention.
Figure 5A shows a top view of the strikethrough plate of Figure 4.
Figure 5B shows a test fluid reservoir of the strikethrough plate of Figure 4.
Figure 6A shows a cross-section view along a longitudinal axis (A-A) of the strikethrough plate of Figure 4.
Figure 6B shows a cross-section view along a lateral axis (B-B) of the strikethrough plate of Figure 4.

DETAILED DESCRIPTION

As used herein, the term "absorbent core" refers to an absorbent core that is has one or more absorbent core layers. Each absorbent core layer is capable acquiring and transporting or retaining fluid.

As used herein, the term "apertured" refers to a nonwoven that has been subjected to a mechanical means that results in weakening patterned locations over a surface of the nonwoven. The weakened locations may be partially or fully open.

As used herein, the term "bicomponent fibers" refers to fibers which have been formed from at least two different polymers extruded from separate extruders but spun together to form one fiber. Bicomponent fibers are also sometimes referred to as conjugate fibers or multicomponent fibers.

As used herein, the term "biconstituent fibers" refers to fibers which have been formed from at least two polymers extruded from the same extruder as a blend. Biconstituent fibers do not have the various polymer components arranged in relatively constantly positioned distinct zones across the cross-sectional area of the fiber and the various polymers are usually not continuous along the entire length of the fiber, instead usually forming fibrils which start and end at random. Biconstituent fibers are sometimes also referred to as multiconstituent fibers.

As used herein, the term "disposable" is describes articles, which are not intended to be laundered or otherwise restored or reused as an article (i.e., they are intended to be discarded after a single use and possibly to be recycled, composted or otherwise disposed of in an environmentally compatible manner). The absorbent article according to the present invention can be for example a sanitary napkin or a panty liner or an adult incontinence article or a baby diaper or a wound dressing. The present invention will be herein described in the context of a typical absorbent article, such as, for example, a sanitary napkin. Typically, such articles can comprise a liquid pervious topsheet, a backsheet and an absorbent core intermediate the topsheet and the backsheet.

As used herein, the term "nonwoven web" refers to a web having a structure of individual fibers or threads which are interlaid, but not in a repeating pattern as in a woven or knitted fabric, which do not typically have randomly oriented fibers. Nonwoven webs or fabrics have been formed from many processes, such as, for example, meltblowing processes, spunbonding processes, spunlacing processes, hydroentangling, airlaying, and bonded carded web processes, including carded thermal bonding. The basis weight of nonwoven fabrics is usually expressed in grams per square meter (gsm). The basis weight of the laminate web is the combined basis weight of the constituent layers and any other added components. Fiber diameters are usually expressed in microns; fiber size can also be expressed in denier, which is a unit of weight per length of fiber. The basis weight of laminate webs suitable for use in an article of the present invention can range from 10 gsm to 100 gsm, 10 gsm to 70 gsm, 10 gsm to 50 gsm, 10 gsm to 40 gsm, or 10 gsm to 30 gsm, specifically including all values within these ranges and any ranges created thereby, depending on the ultimate use of the web.

The disposable absorbent articles, particularly sanitary napkins, sanitary pants, diapers, or training pants, of the present invention provide a mitigated fluid flow over the body facing surface of the article during use once bodily fluid has been expelled onto its surface. In particular, it is envisioned that the articles of the present invention exhibit increased dryness post insult due in large part to the restriction of fluid in a limited area of the product. For the purposes of this disclosure, reference to a sanitary napkin, diaper, disposable absorbent article, or absorbent article will be used. The present invention, however, may be useful in the form of a plurality of disposable absorbent articles including, but not limited to, sanitary napkins, pantiliners, menstrual pads, training pants, etc.

In designing a disposable absorbent article, e.g., a sanitary napkin or diaper, one of the aspects of the product that a designer focuses on is ensuring that there is adequate absorbency. It goes without saying that in order for the article to be perceived as functional, it has to be able to withstand the insults of bodily exudates it encounters. Hand in hand with this primary capability is a less often considered ability of the article to minimize the spread of the bodily exudates post insult on the article's body facing surface. It would, therefore, be desirable to somehow reduce the intermolecular forces between the fluid and the body contacting surface of the article, which is often times referred to as a topsheet. This might be accomplished in various ways. For instance, the surface energy of the topsheet material may be reduced merely by the type of topsheet material that is utilized, e.g., hydrophobic, hydrophilic, or omniphobic, or via the application of hydrophobic or hydrophilic coatings onto such a topsheet. Alternatively, one might mechanically alter the surface topography of the topsheet material of such an article. This could be done by abrading, embossing, or perforating the material.

In contrast to these past approaches, the inventors of the present invention have sought to modify the surface properties of the topsheet material to minimize the spread or flow of any contacting fluids to a substantially smaller area than is typically encountered in common expulsion incidents. This minimized spread or creep is facilitated by an excellent absorption of any contacting fluids by underlying layers which ultimately provides a drier feeling by wearers post insult. The inventors have discovered that a disposable absorbent article of certain character is able to provide these benefits that have not truly been delivered to consumers by prior products in the same way.

Figure 1 shows a disposable absorbent article of the present invention, which in this instance is a sanitary napkin 10. The napkin 10 comprises a generally elongated shape. This shape may take the form of a rectangle, oval, hourglass, offset hourglass (one end is wider than an opposite end and a narrowed mid-section between the ends), etc. The important attribute of the shape of the napkin 10 is that it provides sufficient coverage and protection to a user. Any suitable shape, however, may be utilized. The napkin 10 may be symmetric about a longitudinal axis 50 or asymmetric about the longitudinal axis 50. Similarly, the napkin 10 may be symmetric about a lateral axis 60 or asymmetric about the lateral axis 60. The napkin 10 includes an apertured nonwoven topsheet 20 which further comprises a front portion 26, a rear portion 28, and a central portion 27 disposed therebetween. Each of these portions may be equal in terms of length along an entire length of the topsheet and thereby the napkin. Alternatively, the front and rear portions may be longer than the central portions or the central portion may be longer than each of the front and rear portions.

Figure 2 shows a cross-section of the napkin 10 of Figure 1. The sanitary napkin 10 comprises an apertured nonwoven topsheet 20. The topsheet 20 has a body surface 22 which comes in contact with the body of a user during wear, wherein the body surface has an effective aperture area of greater than 1.8 $mm^2$ and less than 3.5 $mm^2$. The topsheet 20 also has an opposing garment surface 24, which faces away from the body of the user during wear. An absorbent core 30 is disposed beneath the topsheet 20. A backsheet 40 is disposed beneath the absorbent core 30 such that the core 30 is disposed between the topsheet 20 and the backsheet 40. This backsheet 40 is typically the outermost layer of the article and it tends to be the layer which is attached or joined to an undergarment. This attachment or joinder may be effected by application of an adhesive, hook and loop fastening, crimping, ulstrasonic bonding, or heat sealing. It should be noted that additional layers may be included within the napkin, particularly between the topsheet 20 and the backsheet 40 but it should be noted that these layers are separate and apart from the absorbent core 30. Suitable additional layers may include secondary topsheets, acquisition layers, additional distribution layers over and above those which will be discussed below, and other useful layers. In this embodiment, a secondary topsheet 25 is disposed beneath the topsheet 20 and on a body surface 31 of the core 30. In certain embodiments, the secondary topsheet (also known as the "STS") has a greater length and width than the absorbent core 30.

Applicant shall now provide more detailed insight into the individual components of the disposable absorbent articles envisioned herein.

## Topsheet

The aperture nonwoven topsheet 20 of the napkin 10 is positioned adjacent a body surface 31 of the absorbent core 30 and may be joined thereto and to the backsheet 40 by attachment methods such as those well known in the art. Suitable attachment methods are described with respect to joining the backsheet 40 to the absorbent core 30. The topsheet 20 and the backsheet 40 may be joined directly to each other along the sanitary napkin periphery and may be indirectly joined together by directly joining them to the absorbent core 30 or additional optional layers within the product like a secondary topsheet which spans the entire or partial area of the article. This indirect or direct joining may be accomplished by these same attachment methods which are well known in the art.

The topsheet should be compliant, soft feeling, and non-irritating to the wearer's skin. Suitable topsheet materials include a liquid pervious material that is oriented towards and contacts the body of the wearer permitting bodily discharges to rapidly penetrate through it without allowing fluid to flow back through the topsheet to the skin of the wearer. A suitable topsheet can be made of various nonwoven materials. Nonlimiting examples of woven and nonwoven materials suitable for use as the topsheet include fibrous materials made from natural fibers, modified natural fibers, synthetic fibers, or

combinations thereof. These fibrous materials can be either hydrophilic or hydrophobic, but it is preferable that the topsheet be hydrophobic or rendered hydrophobic. As an option, one or more portions of the topsheet can be rendered hydrophilic, by the use of any known method for making topsheets containing hydrophilic components. These portions may lie in one or more of the front, central, or rear regions of the topsheet. Methods describing a process for treating the topsheet with a surfactant are disclosed in U.S. Patents Nos. 4,988,344 and 4,988,345, both issued to Reising et al. on January 29, 1991. The topsheet may have hydrophilic fibers, hydrophobic fibers, or combinations thereof.

The topsheet may comprise one or more layers. In certain embodiments, the topsheet may be a single layer, a dual layer, a triple layer, or even more. Where there is more than one layer of the topsheet, any additional layers are referred to herein sequentially assuming a single layer is a first topsheet layer. That is, subsequent layers are known as second, third, or likewise topsheet layers. In the event of multiple layers of the topsheet, the layers may be joined in a myriad of manners including fusion bonding, gluing, ring rolling, etc. The subsequent layers of the topsheet may also be apertured through from the first topsheet layer in their laminate form.

A particularly suitable topsheet comprises staple length polypropylene fibers having a denier of about 1.5, such as Hercules type 151 polypropylene marketed by Hercules, Inc. of Wilmington, Del. As used herein, the term "staple length fibers" refers to those fibers having a length of at least about 15.9 mm (0.62 inches). Another suitable web for use as a topsheet is a nonwoven that is a 25 gsm 70%/30% polyethylene/polypropylene bicomponent (commercially available from Pegas Nonwovens or Fibertex Nonwovens). Such a web may be overbonded using a pattern formed as a result of mated rollers that provide selectively weakening webs as described in US Patent No. 5,916,661 issued to Benson et al. on June 29, 1999. This patterning mechanism may also be relied on to form apertures in the topsheet. In such instances of the present invention, the mated rollers may be set to a depth of engagement (DOE) suitably tied to the aperture sizes desired. In certain instances, the DOE may be targeted to be from 0.045 to 0.085 inches, with a desirable average DOE being 0.065 approximately. In these instances, there are bond sizes (0.1 inches by 0.010 inches) and spacing (e.g., rows with horizontal spacing of 0.12 inches between bonds and the rows are offset by 0.060 inches with no vertical spacing between the rows). These bonds are called melt stabilized zones and when activated by a ring rolling process form the apertures of the aperture nonwoven.

When the topsheet comprises a nonwoven web, the nonwoven may be produced by any known procedure for making nonwoven webs, nonlimiting examples of which include spunbonding, carding, wet-laid, air-laid, meltblown, needle-punching, mechanical entangling, thermo-mechanical entangling, and hydroentangling. The nonwoven may be compression resistant as described in U.S. Patent No. 7,785,690 issued on August 31, 2010. The nonwoven web may have loops as described in U.S. Patent No. 7,838,099 issued on November 23, 2010.

Other suitable nonwoven materials include low basis weight nonwovens, that is, nonwovens having a basis weight of from about 10 $g/m^2$ to about 40 $g/m^2$. An example of such a nonwoven material is commercially available under the tradename P-8 from Veratec, Incorporation, a division of the International Paper Company located in Walpole, Massachusetts. Other useful nonwovens are described in U.S. Patent Nos. 5,792,404 and 5,665,452.

The topsheet may comprise tufts as described in U.S. Patent No. 8,728,049 issued on May 20, 2014, U.S. Patent No. 7,553,532 issued on June 30, 2009, U.S. Patent No. 7,172,801 issued on February 6, 2007, or U.S. Patent No. 8,440,286 issued on May 14, 2013. The topsheet may have an inverse textured web as described in U.S. Patent No. 7,648,752 issued on January 19, 2010. Tufts are also described in U.S. Patent No. 7,410,683 issued on August 12, 2008.

The topsheet may have a pattern of discrete hair-like fibrils as described in U.S. Patent No. 7,655,176 issued on February 2, 2010 or U.S. Patent No. 7,402,723 issued on July 22, 2008.

The topsheet may comprise one or more structurally modified zones as described in U.S. Patent No. 8,614,365 issued on December 24, 2013. These zones may coincide with one or more of the front, central, and rear portions. The topsheet may have one or more out of plane deformations as described in U.S. Patent No. 8,704,036 issued on April 22, 2014. The primary topsheet may have a masking composition as described in U.S. Patent No. 6,025,535 issued on February 15, 2000.

Another suitable topsheet or a topsheet combined with a secondary topsheet may be formed from a three-dimensional substrate as detailed in a U.S. patent application serial no. 15/453,997 filed on March 9, 2017 in the name of Jill M. Orr. This three-dimensional substrate has a first surface, a second surface, land areas and also comprises three-dimensional protrusions extending outward from the second surface of the three-dimensional substrate, wherein the three-dimensional protrusions are surrounded by the land areas. The substrate is a laminate comprising at least two layers in a face to face relationship, the second layer is a tissue layer facing outward from the second surface of the three-dimensional substrate, and the tissue layer comprises at least 80% pulp fibers by weight of the tissue layer.

The topsheet may comprise one or more layers, for example, a spunbond-meltblown-spunbond (SMS) material. The apertured topsheet may have any suitable three-dimensional features, and/or may have a plurality of embossments (e.g., a bond pattern). The aperturing of the topsheet may formed by overbonding a material and then rupturing the overbonds through ring rolling as disclosed in U.S. Patent No. 5,628,097, to Benson et al., issued on May 13, 1997. Additional lateral extensibility in any of the topsheet and/or the backsheet and intermediate layers, (which in combination

may be referred to as a "chassis" of the product) may be provided in a variety of ways. For example, either the topsheet or backsheet may be pleated by any of many known methods. Alternatively, all or a portion of the chassis, including the topsheet and backsheet, may be made of a formed web material or a formed laminate of web materials like those described in U.S. Patent No. 5,518,801 issued on 21 May 1996 to Chappell et al. Such a formed web material includes distinct laterally extending regions in which the original material has been altered by embossing or another method of deformation to create a pattern of generally longitudinally oriented alternating ridges and valleys. The formed web material also includes laterally extending unaltered regions located between the laterally extending altered regions.

The aperturing of the topsheet may be effected by varying formation means which impart weakened patterned locations therein. Such formation means known for deforming a generally planar fibrous web into a three-dimensional structure are utilized in the present invention to modify as-made absorbent materials into absorbent materials having relatively higher permeability without a significant corresponding decrease in capillary pressure. Formation means may comprise a pair of inter-meshing rolls, typically steel rolls having inter-engaging ridges or teeth and grooves. It is, however, contemplated that other means for achieving formation can be utilized, such as the deforming roller and cord arrangement disclosed in US Patent Publication 2005/0140057, published June 30, 2005. Therefore, all disclosure of a pair of rolls herein is considered equivalent to a roll and cord, and a claimed arrangement reciting two inter-meshing rolls is considered equivalent to an inter-meshing roll and cord where a cord functions as the ridges of a mating inter-engaging roll. In one embodiment, the pair of intermeshing rolls of the instant invention can be considered as equivalent to a roll and an inter-meshing element, wherein the inter-meshing element can be another roll, a cord, a plurality of cords, a belt, a pliable web, or straps. Likewise, other known formation technologies, such as creping, necking/consolidation, corrugating, embossing, button break, hot pin punching, and the like are believed to be able to produce absorbent materials having some degree of relatively higher permeability without a significant corresponding decrease in capillary pressure. Formation means employing rolls include "ring rolling", a "SELF" or "SELF'ing" process, in which SELF stands for Structural Elastic Like Film, as "micro-SELF", and "rotary knife aperturing" (RKA) which are both described in US Patent No. 7,935,207 issued to Zhao et al. on May 3, 2011.

Suitable aperture patterns and mechanisms for effecting them are also detailed in US Patent Publications 2016/0129661 and 2016/0129662 published on May 12, 2016 in the name of Aurora et al. The apertures of the nonwoven topsheet may be of a variety of shapes including, but not limited to, rectangular, circular, oblong, triangular, slitted, etc. One important aspect of the topsheet is its effective aperture area of the body surface. Without being limited by theory, the inventors have surmised that the apertures may range in size across the body surface of the topsheet but it is critical that the topsheet exhibit an effective aperture area of less than 3.5 $mm^2$. In particular, the effective aperture area is greater than 1.8 $mm^2$ and less than 3.5 $mm^2$. Preferably, the effective aperture area is greater than 1.9 $mm^2$, 2 $mm^2$, or 2.1 $mm^2$ and preferably less than 3.4 $mm^2$, 3.2 $mm^2$, 3 $mm^2$, 2.8 $mm^2$, 2.6 $mm^2$, or 2.4 $mm^2$. This is the case because if a collection of apertures in one or more of the portions of the topsheet are too narrow or small, when taken together, they will not permit suitably useful acquisition of insulted fluids by the article. Such a configuration will likely cause pooling of insulted fluids on the body surface of the topsheet, which is definitely not desirable. On the other hand, if a collection of apertures is too large or sizable, the topsheet becomes ineffective in acting as a barrier layer to the wearer from the fluid which has been absorbed by any underlying layers. This effect might ultimately result in a wet feeling to the wearer post insult. Moreover, in conjunction with the requisite effective aperture area, it is ideal that the topsheet also exhibit an average % effective area of less than or equal to 20%, 18%, 16%, 14%, 12%, or 11%. This ensures that the area open to the underlying layers, e.g., a secondary topsheet or acquisition layer, in one or more of the front, central, or rear portions of the topsheet, is not too voluminous such that a concentration of apertures permits maintenance of a barrier between the wearer and the absorbed fluid in the article post insult.

The overall configuration of the topsheet including the below described application of a surface modifying composition and aperturing result in a topsheet that, once contacted with fluid, e.g., artificial menstrual fluid (AMF), has an heightened ability to deflect such fluid to underlying layers in the disposable absorbent article or product. This ability to contain and then deflect fluid via absorption by underlying layers is evidenced in a variety of ways. For instance, the disposable absorbent articles of the present invention, exhibit a reduced Interfacial Fluid Area of the topsheet and an underlying layer (typically an STS or an acquisition layer). The Interfacial Fluid Area is less than 300 $mm^2$, 280 $mm^2$, 260 $mm^2$, or 240 $mm^2$. The measurement of the Interfacial Fluid Area is described below in the Test Methods section. The present articles also exhibit a Free Fluid Acquisition Time of less than 30 seconds. The Free Fluid Acquisition Time is indicative of the article's overall ability to draw fluid away from a skin surface of a wearer of the article. The quicker the Free Fluid Acquisition Time, the better performing or drier feeling the article is perceived by a wearer. For instance, the Free Fluid Acquisition Time of the article may be less than 28s, 26s, 24s, 22s, 20s, 18s, 16s, 14s, 12s, 10s, 8s, or even 6s. There must, however, be a balance of this measurement with others. For instance, the topsheet of the present articles also exhibit a reduced or minimal Topsheet Stain Area on the body surface post insult, once the topsheet is removed from the article. This removed Topsheet Stain Area is intended to be smaller in comparison to that exhibited on untreated topsheets or topsheets treated with compositions different than those discussed and claimed herein. The Topsheet Stain Area is less than 30 $mm^2$, 25 $mm^2$, 20 $mm^2$, 15 $mm^2$, 13 $mm^2$, 12 $mm^2$, or 10 $mm^2$. The method for

measuring this resultant Topsheet Stain Area on the removed topsheet is detailed in the Test Methods section below. The Fiber Roughness value is also another measurement that is useful herein to quantify the increased change in topography of the surface area of the nonwoven fibers as a result of application of the surface modification composition. Ideally, the Fiber Roughness value is increased versus an untreated nonwoven. It is envisioned that the Fiber Roughness value of the present invention would range from 1 μm to about 3.5 μm, 3μm, 2.5 μm, or even 2 μm. This method is detailed in the Test Methods section below. The final measurement that the inventors have identified useful in distinguishing the articles of the present invention from other disposable absorbent articles is that of Stain Chroma Value. This measurement is indicative of the intensity of the stain post insult that is observed by a wearer or user. The method for determining this value is also detailed below in the Test Methods section.

Surface Modifying Composition

The articles of the present invention comprise a surface modifying composition 35 disposed on the body surface of the topsheet. This composition comprises hydrophobic particles and a hydrophobic binder in a binder to particles ratio of from 4:1 to 1:4, 2:1 to 1:2, or even 1: 1. Such ratios are measured on a weight basis. These compositions are applied on the topsheet in an amount of 0.1 gsm, 0.15 gsm, or 0.2 gsm to 0.5 gsm, 0.75 gsm, 1 gsm, 1.5 gsm, 2 gsm, 2.5 gsm, or even 3 gsm on one or more of the front portion, central portion, and rear portions of the body surface of that topsheet. The compositions provided in these ratios of binder to particles provide optimal conditions for the body surface 22 of the topsheet 20 to exhibit the minimized spreadability and adhesion of fluids thereon.

The inventors have discovered that the application of this composition to the topsheet drives liquid that is introduced to the respective article or product to a reduced area where such fluid is then subsequently absorbed by the underlying layers. In particular, the surface modifying composition results in a reduced spread area on the topsheet as well as increased absorption by the underlying layers such that the observed Stain Area on the topsheet upon removal is measured at less than 30 $mm^2$. In other embodiments, this observed Topsheet Stain Area is less than 25 $mm^2$, 20 $mm^2$, 15 $mm^2$, 10 $mm^2$, or less than 8 $mm^2$. In contrast, when the ratio of the binder to particles is outside of the ratio 4:1 to 1:4, the same observed Stain Area is substantially increased. This result is highly undesirable by a consumer or wearer.

The surface modifying composition may be disposed on the body surface of the topsheet in a variety of ways including, but not limited to, slot coating, spraying, wetting, dipping, printing, or any other application method that is suitable and known in the art. The composition may be applied to one or more areas within the front, central, and/or rear portions. For instance, in certain embodiments and product types, it may only be necessary to treat the central portion of the product in the event the napkin is intended for wear during the day time by adult consumers. In contrast, where the product may be a training pant, a toddler diaper, or adult overnight sanitary napkin, it would be advantageous to optimize the topsheet's performance by applying the surface modifying composition along a full length of the product such that the front, central, and rear portions of the topsheet are treated.

Suitable hydrophobic particles are selected from the group consisting of hydrophobically modified silicas, modified polyacrylates, polymethacrylates, acrylate-vinyl acetate copolymers, styrene acrylic copolymers, carboxylated styrene butadiene copolymers, and combinations thereof. Exemplary particles useful in the present invention are listed below in Table 1. These particles may be used individually or in combination with each other or other similarly performing particulate materials that combine to provide the requisite surface properties to the nonwoven.

Table 1. Particle Listing

| **Particle Type** | **Trade Name** | **Chemistry** | **Manufacturer** | **Manufacturer Location** |
|---|---|---|---|---|
| R812 | Aerosil R 812<br>Aerosil R 812s | Fumed Silica treated with HDMS | Evonik Industries | Essen, Germany |
| R816 | Aerosil R 816 | Fumed Silica treated with a hexadecylsilane | Evonik Industries | Essen, Germany |
| Acronal NX 4612 | Acronal NX 4612 | Acrylic polymers | BASF | Charlotte, NC, USA |
| Acronal NX 5818 | Acronal NX 5818 | Styrene acrylic polymer | BASF | Charlotte, NC, USA |

With regard to the hydrophobic binder, such a material may be selected from the group consisting of polydimethylsiloxanes, aminofunctionalized silicones, dimethyl silicones, polyisobutylane, hydrogenated triglycerides, hydrogenated oil waxes, soy wax, polyols, and combinations thereof.

In one embodiment of the present invention, the hydrophobic binder is an organopolysiloxane having the formula:

$$M_wD_xT_yQ_z$$

wherein:

M is selected from the group consisting of $[SiR_1R_2R_3O_{1/2}]$, $[SiR_1R_2G_1O_{1/2}]$, $[SiR_1G_1G_2O_{1/2}]$, $[SiG_1G_2G_3O_{1/2}]$, and combinations thereof;
D is selected from the group consisting of $[SiR_1R_2O_{2/2}]$, $[SiR_1G_1O_{2/2}]$, $[SiG_1G_2O_{2/2}]$, and combinations thereof;
T is selected from the group consisting of $[SiR_1O_{3/2}]$, $[SiG_1O_{3/2}]$, and combinations thereof;
Q = $[SiO_{4/2}]$;
w is an integer from 1 to about (2+y+2z);
x is an integer from about 5 to about 15,000;
y is an integer from 0 to about 98;
z is an integer from 0 to about 98;
$R_1$, $R_2$ and $R_3$ are each independently selected from the group consisting of H, OH, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $C_1$-$C_{32}$ alkoxy, $C_1$-$C_{32}$ substituted alkoxy, $C_1$-$C_{32}$ alkylamino, and $C_1$-$C_{32}$ substituted alkylamino;
and wherein at least one of M, D, and T incorporates at least one moiety $G_1$, $G_2$ or $G_3$ and
$G_1$, $G_2$, and $G_3$ are same or different moieties each of which has the formula:

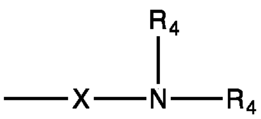

or

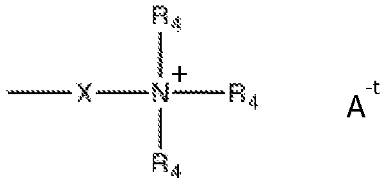

wherein:
X comprises a divalent radical selected from the group consisting of $C_1$-$C_{32}$ alkylene, $C_1$-$C_{32}$ substituted alkylene, optionally interrupted with a hetero atom selected from the group consisting of P, N and O, $C_5$-$C_{32}$ or $C_6$-$C_{32}$ arylene, $C_5$-$C_{32}$ or $C_6$-$C_{32}$ substituted arylene, $C_6$-$C_{32}$ arylalkylene, $C_6$-$C_{32}$ substituted arylalkylene, $C_1$-$C_{32}$ alkyleneamino, $C_1$-$C_{32}$ substituted alkyleneamino, ring-opened epoxide and ring-opened glycidyl;
N is a nitrogen atom;
each $R_4$ and each is independently selected from the group consisting of H,

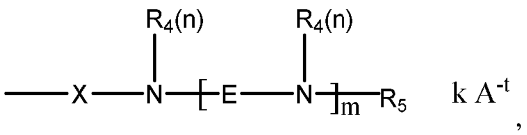

$C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, and $C_6$-$C_{32}$ substituted alkylaryl;
wherein
E comprises the same or different divalent radicals selected from the group consisting of $C_1$-$C_{32}$ alkylene, $C_1$-$C_{32}$ substituted alkylene, alkylene, optionally interrupted with a hetero atom selected from the group consisting of P, N and O, $C_5$-$C_{32}$ or $C_6$-$C_{32}$ arylene, $C_5$-$C_{32}$ or $C_6$-$C_{32}$ substituted arylene, $C_6$-$C_{32}$ arylalkylene, $C_6$-$C_{32}$ substituted arylalkylene, $C_1$-$C_{32}$ alkoxy, $C_1$-$C_{32}$ substituted alkoxy, $C_1$-$C_{32}$ alkyleneamino, $C_1$-$C_{32}$ substituted alkyleneamino, ring-opened epoxide and ring-opened glycidyl;
$R_5$ is independently selected from the group consisting of H, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, and $C_6$-$C_{32}$ substituted alkylaryl; or $R_5$ comprises one or more $M_wD_xT_yQ_z$ moieties;
and wherein at least one R5 comprises at least one $M_wD_xT_yQ_z$ moiety;

and wherein

m is an integer independently selected from 2 to 100,
n is an integer independently selected from 1 or 2,
and when an organopolysiloxane portion of a moiety $G_1$, $G_2$, $G_3$ is positively charged, $A^{-t}$ is a suitable charge balancing anion or anions such that the total charge of all occurrences of the charge-balancing anion or anions, $A^{-t}$ and $kA^{-t}$, is equal to and opposite from the net charge on the organopolysiloxane portions of the moiety $G_1$, $G_2$ or $G_3$.

It would be appreciated by one of ordinary skill in the art that:

A is an anionic counter ion to the positively charge organopolysiloxane,
t is the charge on any individual counter ion, and
k is the coefficient of any such counterion

so that the net charge of the positively charge organopolysiloxane and the sum total of the counter ions is neutral.

In one embodiment, the blocky cationic organopolysiloxane has the formula:

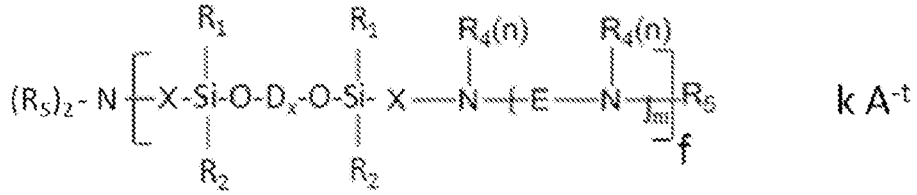

or

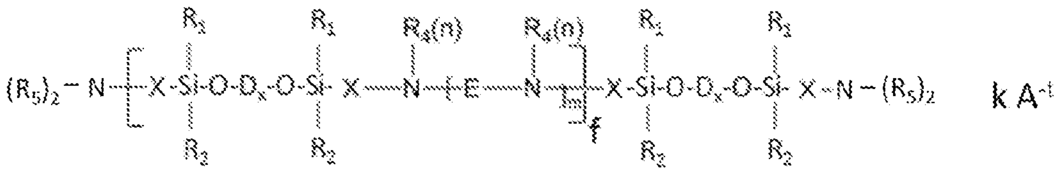

or

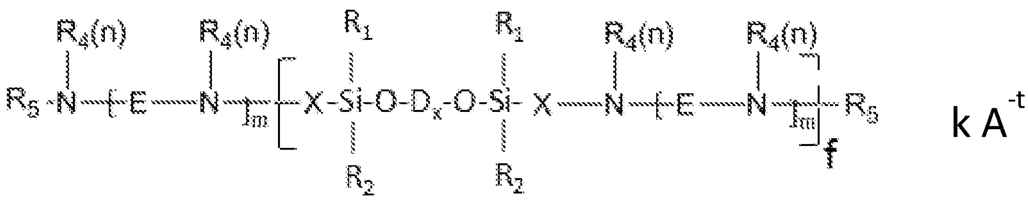

wherein

D is $[SiR_1R_2O_{2/2}]$,
x is an integer independently selected from about 40 to about 1000,
$R_1$, $R_2$ and are independently selected from the group consisting of H, OH, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $C_1$-$C_{32}$ alkoxy, $C_1$-$C_{32}$ substituted alkoxy,
wherein

X comprises a divalent radical selected from the group consisting of $C_1$-$C_{32}$ alkylene, $C_1$-$C_{32}$ substituted alkylene, optionally interrupted with a hetero atom selected from the group consisting of P, N and O, $C_5$-$C_{32}$ or $C_6$-$C_{32}$ arylene, $C_5$-$C_{32}$ or $C_6$-$C_{32}$ substituted arylene, $C_6$-$C_{32}$ arylalkylene, $C_6$-$C_{32}$ substituted arylalkylene, $C_1$-$C_{32}$ alkyleneamino, $C_1$-$C_{32}$ substituted alkyleneamino, ring-opened epoxide and ring-opened glycidyl;

$R_4$ is independently selected from the group consisting of H,

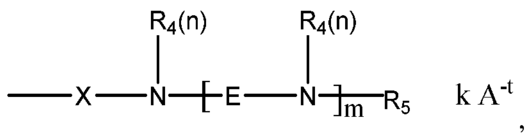

$C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, and $C_6$-$C_{32}$ substituted alkylaryl;

N is a nitrogen atom;

$R_5$ is, independently, selected from the group consisting of H, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, and $C_6$-$C_{32}$ substituted alkylaryl optionally interrupted with a hetero atom selected from the group consisting of P, N and O, $C_1$-$C_{32}$ alkoxy, $C_1$-$C_{32}$ substituted alkoxy, $C_1$-$C_{32}$ alkylamino, or $C_1$-$C_{32}$ substituted alkylamino;

E comprises same or different divalent radicals selected from the group consisting of $C_1$-$C_{32}$ alkylene, $C_1$-$C_{32}$ substituted alkylene, alkylene, optionally interrupted with a hetero atom selected from the group consisting of P, N and O, $C_5$-$C_{32}$ or $C_6$-$C_{32}$ arylene, $C_5$-$C_{32}$ or $C_6$-$C_{32}$ substituted arylene, $C_6$-$C_{32}$ arylalkylene, $C_6$-$C_{32}$ substituted arylalkylene, $C_1$-$C_{32}$ alkoxy, $C_1$-$C_{32}$ substituted alkoxy, $C_1$-$C_{32}$ alkyleneamino, $C_1$-$C_{32}$ substituted alkyleneamino, ring-opened epoxide and ring-opened glycidyl;

m is an integer independently selected from 2 to 100,

n is an integer independently selected from 1 or 2,

f is an integer from 2 to about 50, and

$A^{-t}$ is a suitable charge balancing anion or anions such that the total charge of all occurrences of the charge-balancing anion or anions, $A^{-t}$ and $kA^{-t}$, is equal to and opposite from the net charge on the blocky cationic organopolysiloxane.

In one embodiment, $A^{-t}$ is selected from the group consisting of Cl-, Br-, I-, methylsulfate, toluene sulfonate, carboxylate, phosphate, hydroxide, acetate, formate, carbonate, nitrate, and combinations thereof.

In one embodiment that blocky cationic organopolysiloxane has the structure:

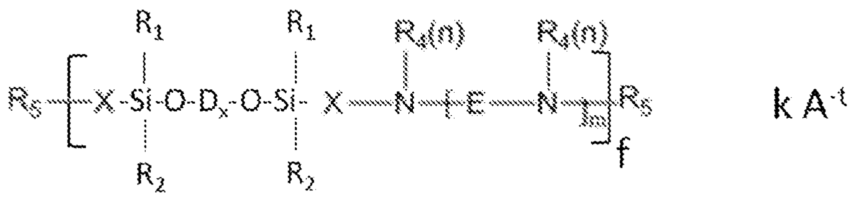

or

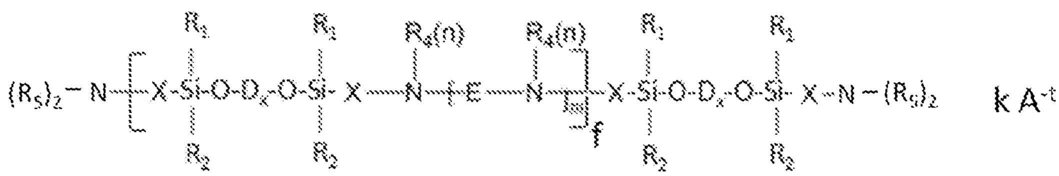

or

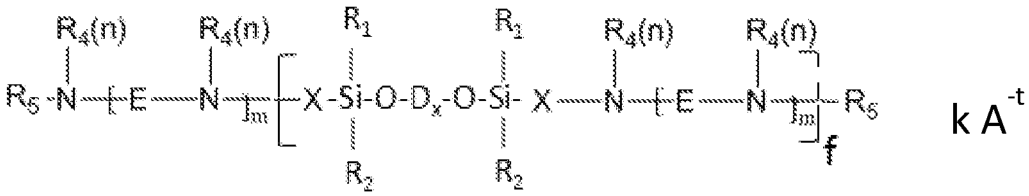

wherein

D is $[SiR_1R_2O_{2/2}]$,

x is an integer independently selected from about 40 to about 1000,

$R_1$, $R_2$ and are independently selected from the group consisting of H, OH, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, $C_6$-$C_{32}$ substituted alkylaryl, $C_1$-$C_{32}$ alkoxy, $C_1$-$C_{32}$ substituted alkoxy,

wherein
X comprises a divalent radical selected from the group consisting of $C_1$-$C_{32}$ alkylene, $C_1$-$C_{32}$ substituted alkylene, optionally interrupted with a hetero atom selected from the group consisting of P, N and O, $C_5$-$C_{32}$ or $C_6$-$C_{32}$ arylene, $C_5$-$C_{32}$ or $C_6$-$C_{32}$ substituted arylene, $C_6$-$C_{32}$ arylalkylene, $C_6$-$C_{32}$ substituted arylalkylene, $C_1$-$C_{32}$ alkyleneamino, $C_1$-$C_{32}$ substituted alkyleneamino;

$R_4$ is independently selected from the group consisting of H,

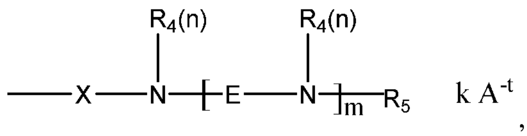

$C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, and $C_6$-$C_{32}$ substituted alkylaryl;

N is a nitrogen atom;

$R_5$ is, independently, selected from the group consisting of H, $C_1$-$C_{32}$ alkyl, $C_1$-$C_{32}$ substituted alkyl, $C_5$-$C_{32}$ or $C_6$-$C_{32}$ aryl, $C_5$-$C_{32}$ or $C_6$-$C_{32}$ substituted aryl, $C_6$-$C_{32}$ alkylaryl, and $C_6$-$C_{32}$ substituted alkylaryl, optionally interrupted with a hetero atom selected from the group consisting of P, N and O, $C_1$-$C_{32}$ alkoxy, $C_1$-$C_{32}$ substituted alkoxy, $C_1$-$C_{32}$ alkylamino, $C_1$-$C_{32}$ substituted alkylamino;

E comprises same or different divalent radicals selected from the group consisting of $C_1$-$C_{32}$ alkylene, $C_1$-$C_{32}$ substituted alkylene, alkylene, optionally interrupted with a hetero atom selected from the group consisting of P, N and O, $C_5$-$C_{32}$ or $C_6$-$C_{32}$ arylene, $C_5$-$C_{32}$ or $C_6$-$C_{32}$ substituted arylene, $C_6$-$C_{32}$ arylalkylene, $C_6$-$C_{32}$ substituted arylalkylene, $C_1$-$C_{32}$ alkoxy, $C_1$-$C_{32}$ substituted alkoxy, $C_1$-$C_{32}$ alkyleneamino, $C_1$-$C_{32}$ substituted alkyleneamino, ring-opened epoxide and ring-opened glycidyl;

m is an integer independently selected from 2 to 100,

n is an integer independently selected from 1 or 2,

f is an integer from 2 to about 50, and
$A^{-t}$ is a suitable charge balancing anion or anions such that the total charge of all occurrences of the charge-balancing anion or anions, $A^{-t}$ and $kA^{-t}$, is equal to and opposite from the net charge on the blocky cationic organopolysiloxane.

In terms of suitable polyols that are useful as hydrophobic binders, one can look to metathesized unsaturated polyol esters, including Elevance Smooth CS-110, can be obtained from Elevance Renewable Sciences, Inc., of

Woodridge, Ill. USA. or from Dow Corning of Midland Michigan USA under the name DOW CORNING® HY-3050 SOY WAX. Additional exemplary metathesized unsaturated polyol esters and their starting materials are set forth in U.S. Patent Publications 2009/0220443 A1, 2013/0344012 A1 and 2014/0357714 A1. A metathesized unsaturated polyol ester refers to the product obtained when one or more unsaturated polyol ester ingredient(s) are subjected to a metathesis reaction. Metathesis is a catalytic reaction that involves the interchange of alkylidene units among compounds containing one or more double bonds (i.e., olefinic compounds) via the formation and cleavage of the carbon-carbon double bonds. Metathesis may occur between two of the same molecules (often referred to as self-metathesis) and/or it may occur between two different molecules (often referred to as cross-metathesis).

Additional exemplary hydrophobic binders that may be employed in the present invention are listed below in Table 2.

Table 2. Hydrophobic Binder Listing

| Binder Type | Trade Name | Chemistry | Manufacturer | Manufacturer Location |
|---|---|---|---|---|
| AP6087 | AP-6087 | Aminofunctional Siloxane | Shin-Etsu Chemical Co | Tokyo, Japan |
| WR1300 | WR1300 | Aminosilicone | Wacker | Munich, Germany |
| CS 110 | Elevance Smooth CS-110 | Hydrogenated soy polyglycerides and C15-23 alkane | Elevance | Woodridge, IL |
| Soy Wax | Soy Wax 350 | Hydrogenated soybean oil wax | Koster Keunen | Watertown, CT |
| Y-12528 | Y-12528 | Organomodified Amino Silicone | Momentive | Waterford, NY |
| DMS-A15 | DMS-A15 | Reactive Aminopropyl Terminated PDMS | Gelest | Morrisville, PA |
| DMS-A31 | DMS-A31 | Reactive Aminopropyl Terminated PDMS | Gelest | Morrisville, PA |
| KF867 | KF867 | Reactive amino modified silicone | Shin-Etsu Chemical Co | Tokyo, Japan |

Tables 1 and 2 are not comprehensive but are indicative of hydrophobic particles and hydrophobic binders that have been found useful in the present invention.

The surface modifying composition may further comprise a solvent to aid in the suspension of the hydrophobic particles and the hydrophobic binder. Suitable solvents include, but are not limited to, water, ethanol, isopropyl alcohol, propylene glycol n-butyl ether, or any other volatile solvents. The resultant composition, once applied to the topsheet via spraying, dipping, wetting, printing, imparts a rougher topographical character to the topsheet. This modified topography of the topsheet results in the topsheet exhibiting a Fiber Roughness of about 1 μm to about 3.5 μm. In other embodiments, the Fiber Roughness is from 1 μm to 3 μm, 1 μm to 2.5 μm, or 1 to 2 μm.

Backsheet

The backsheet 40 of the chassis may be positioned adjacent a garment-facing surface of the absorbent core 30 and may be joined thereto by attachment methods (not shown) such as those well known in the art. For example, the backsheet 40 may be secured to the absorbent core 30 by a uniform continuous layer of adhesive, a patterned layer of adhesive, or an array of separate lines, spirals, or spots of adhesive. Alternatively, the attachment methods may comprise using heat bonds, pressure bonds, ultrasonic bonds, dynamic mechanical bonds, or any other suitable attachment methods or combinations of these attachment methods as are known in the art. Forms of the present disclosure are also contemplated wherein the absorbent core 30 is not joined to the backsheet 40, the topsheet 20, or both.

The backsheet 40 may be impervious, or substantially impervious, to liquids (e.g., urine) and may be manufactured from a thin plastic film, although other flexible liquid impervious materials may also be used. As used herein, the term "flexible" refers to materials which are compliant and will readily conform to the general shape and contours of the human body. The backsheet 40 may prevent, or at least inhibit, the exudates absorbed and contained in the absorbent core 30 from wetting articles of clothing which contact the sanitary napkin 10 such as undergarments. In some instances, the backsheet 40 may permit vapors to escape from the absorbent core 30 (i.e., is breathable) while in other instances

the backsheet 40 may not permit vapors to escape (i.e., non-breathable). Thus, the backsheet 40 may comprise a polymeric film such as thermoplastic films of polyethylene or polypropylene. A suitable material for the backsheet 40 is a thermoplastic film having a thickness of from about 0.012 mm (0.5 mil) to about 0.051 mm (2.0 mils), for example. Any suitable backsheet known in the art may be utilized with the present invention.

The backsheet 40 acts as a barrier to any absorbed bodily fluids that may pass through the absorbent core 30 to the garment surface thereof with a resulting reduction in risk of staining undergarments or other clothing. Further, the barrier properties of the backsheet permit manual removal, if a wearer so desires, of the interlabial absorbent article with reduced risk of hand soiling. A preferred material is a soft, smooth, compliant, liquid and vapor pervious material that provides for softness and conformability for comfort, and is low noise producing so that movement does not cause unwanted sound.

The backsheet 40 may comprise a wet laid fibrous assembly having a temporary wet strength resin incorporated therein as described in US Patent No. 5,885,265 issued to Osborn on March 23, 1999. The backsheet 40 may further be coated with a water resistant resinous material that causes the backsheet to become impervious to bodily fluids without impairing the spreading of adhesive materials thereon.

Another suitable backsheet material is a polyethylene film having a thickness of from about 0.012 mm (0.5 mil) to about 0.051 mm (2.0 mils). The backsheet may be embossed and/or matte finished to provide a more clothlike appearance. Further, the backsheet may permit vapors to escape from the absorbent core (i.e., the backsheet is breathable) while still preventing body fluids from passing through the backsheet. A suitable material for the backsheet is a microporous polyethylene film which is available from Tredegar Corporation, Virginia, USA, under Code No. XBF-1 12W.

For a stretchable but non-elastic backsheet, one material that may be used is a hydrophobic, stretchable, spun laced, non-woven material that is breathable, i.e. permeable to water vapor and other gases, and has a basis weight of from about 30 to 40 g/$m^2$, formed of polyethylene terephthalate or polypropylene fibers.

For an elastic backsheet, an elastic film sold under the trade mark EXX500 by Exxon Corporation may be used. This non-breathable film is formed from an elastomeric base composition consisting of a styrene block copolymer. Another material which can be used for an elastic backsheet is a plastic film that has been subjected to a process that provides it with elastic-like properties without attaching elastic strands to the film, and may, for example, comprise a formed film made as detailed in US Patent Nos. 4,342,314 issued to Radel et al. and 4,463,045 to Ahr et al., respectively.

Suitable breathable backsheets for use herein include all breathable backsheets known in the art. There are two types of breathable backsheets. Single layer breathable backsheets which are breathable and impervious to liquids and backsheets having at least two layers, which in combination provide both breathability and liquid imperviousness. Suitable single layer breathable backsheets for use herein include those described for example in U.S. Pat. Nos. 4,695,422, 4,839,216, 4,591,523, 3 989 867, 3,156,242 and WO 97/24097.

The backsheet may have two layers: a first layer comprising a gas permeable aperture formed film layer and a second layer comprising a breathable microporous film layer as described in US Patent No. 6,462,251. Suitable dual or multi-layer breathable backsheets for use herein include those exemplified in U.S. Pat. Nos. 3,881,489, 4,341,216, 4,713,068, 4,818,600, and European Patent Publications 203821, 710471, 710 472, and 793952.

The backsheet may be a relatively hydrophobic 18 grams per square meter (gsm) spunbonded nonwoven web of 2 denier polypropylene fibers. The backsheet may also be a laminate.

The backsheet may be vapor permeable as described in US Patent No. 6,623,464 to Bewick-Sonntag issued September 23, 2003 or US Patent No. 6,664439 to Arndt issued December 16, 2003. The backsheet can be formed from any vapor permeable material known in the art. The backsheet can be a microporous film, an apertured formed film, or other polymer film that is vapor permeable, or rendered to be vapor permeable.

In other embodiments, the backsheet may be a nonwoven web having a basis weight between 20 gsm and 50 gsm. In one embodiment, the backsheet is a relatively hydrophobic 23 gsm spunbonded nonwoven web of 4 denier polypropylene fibers available from Fiberweb Neuberger, under the designation F102301001. The backsheet may be coated with a non-soluble, liquid swellable material as described in US Patent No. 6,436,508 issued to Ciammaichella on August 20, 2002.

The backsheet 40 has a garment surface 42 and an opposing body surface 41. The garment surface of the backsheet comprises a non-adhesive area and an adhesive area. The adhesive area may be provided by any conventional means. Pressure sensitive adhesives have been commonly found to work well for this purpose.

Absorbent Core

The absorbent core 30 of the present invention may comprise any suitable shape including but not limited to an oval, a discorectangle, a rectangle, an asymmetric shape, and an hourglass. For example, in some forms of the present invention, the absorbent core 30 may comprise a contoured shape, e.g. narrower in the intermediate region than in the end regions. As yet another example, the absorbent core may comprise a tapered shape having a wider portion in one end region of the pad which tapers to a narrower end region in the other end region of the pad. The

absorbent core 30 may comprise varying stiffness in the MD and CD.

The absorbent core 30 may comprise one or more absorbent layers. In certain embodiments, there are two absorbent layers where there is a first absorbent layer and a second absorbent layer adjacent to the first absorbent layer. These materials are generally compressible, conformable, non-irritating to the wearer's skin, and capable of absorbing and retaining liquids such as urine and other certain body exudates including menses. The first absorbent layer may comprise a first layer of absorbent material, which may be 100% or less of superabsorbent polymer (SAP), such as 85% to 100% SAP, 90% to 100% SAP, or even 95% to 100% SAP, specifically including all 0.5% increments within the specified ranges and all ranges formed therein or thereby. The second absorbent layer may comprise a second layer of absorbent material, which may also be 100% or less of SAP (including the ranges specified above). Alternatively, the first and/or second absorbent layer may each comprise a combination of cellulose, commuted wood pulp, or the like in combination with SAP. Additionally, the absorbent core may also be comprised solely of cellulosic material (also known as "airlaid" or "airfelt") as the absorbent material. The absorbent core 30 may also comprise a carrier layer for either or both of the first and second absorbent layers. This carrier layer may be a nonwoven web as well which may be apertured or not. The absorbent core 30 may also comprise a fibrous thermoplastic adhesive material at least partially bonding each layer of the absorbent material to its respective material. These SAPs are also known as absorbent gelling materials or AGMs.

The absorbent core 30 may comprise one or more pockets. The one or more pockets may be provided in addition to the one or more channels or instead of the one or more channels. The pockets may be areas in the absorbent core that are free of, or substantially free of absorbent material, such as SAP (including the ranges specified above). Other forms and more details regarding channels and pockets that are free of, or substantially free of absorbent materials, such as SAP, within absorbent cores are discussed in greater detail in U.S. Patent Application Publication Nos. 2014/0163500, 2014/0163506, and 2014/0163511, each published on June 12, 2014.

The configuration and construction of the absorbent core 30 may vary (e.g., the absorbent core 30 may have varying caliper zones, a hydrophilic gradient, a superabsorbent gradient, or lower average density and lower average basis weight acquisition zones). Further, the size and absorbent capacity of the absorbent core 30 may also be varied to accommodate a variety of wearers. However, the total absorbent capacity of the absorbent core 30 should be compatible with the design loading and the intended use of the sanitary napkin or any other disposable absorbent article.

In some forms of the present invention, the absorbent core 30 may comprise a plurality of multi-functional layers in addition to the first and second absorbent layers. For example, the absorbent core 30 may comprise a core wrap (not shown) useful for enveloping the first and second absorbent layers and other optional layers. The core wrap may be formed by two nonwoven materials, substrates, laminates, films, or other materials. The core wrap may only comprise a single material, substrate, laminate, or other material wrapped at least partially around itself.

The absorbent core 30 may comprise one or more adhesives, for example, to help immobilize any superabsorbent gelling material or other absorbent materials that might be present in the core.

Absorbent cores comprising relatively high amounts of SAP with various core designs are disclosed in U.S. Pat. No. 5,599,335 to Goldman et al., EP 1,447,066 to Busam et al., WO 95/11652 to Tanzer et al., U.S. Pat. Publ. No. 2008/0312622A1 to Hundorf et al., and WO 2012/052172 to Van Malderen. These designs may be used to configure the first and second superabsorbent layers. Alternate core embodiments are also described in U.S. Pat. No. 4,610,678 issued to Weisman et al., on Sep. 9, 1986; U.S. Pat. No. 4,673,402 issued to Weisman et al., on Jun. 16, 1987; U.S. Pat. No. 4,888,231 issued to Angstadt on Dec. 19, 1989; and U.S. Pat. No. 4,834,735 issued to Alemany et al., on May 30, 1989. The absorbent core may further comprise additional layers that mimic a dual core system containing an acquisition/distribution core of chemically stiffened fibers positioned over an absorbent storage core as detailed in U.S. Pat. No. 5,234,423 issued to Alemany et al., on Aug. 10, 1993; and in U.S. Pat. No. 5,147,345.

The SAPs of the present invention may have various make ups. One highly preferred type of hydrogel-forming, absorbent gelling material is based on the hydrolyzed polyacids, especially neutralized polyacrylic acid. Hydrogel-forming polymeric materials of this type are those which, upon contact with fluids (i.e., liquids) such as water or body fluids, imbibe such fluids and thereby form hydrogels. In this manner, fluid discharged into the fluid absorbent structures herein can be acquired and held. These preferred superabsorbent polymers will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. The hydrolyzed polyacrylic acid grafted starch materials are of this latter type. Thus, the preferred superabsorbent polymers include hydrolyzed polyacrylonitrile grafted starch, hydrolyzed polyacrylate grafted starch, polyacrylates, maleic anhydride-iso-butylene copolymers and combinations thereof. Especially preferred superabsorbent polymers are the hydrolyzed polyacrylates and hydrolyzed polyacrylate grafted starch.

Whatever the nature of the polymer components of the preferred superabsorbent polymers, such materials will in general be slightly cross-linked. Cross-linking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example: (1) compounds having at least two polymerizable double bonds; (2) compounds having at least one polymerizable double bond and at least one functional group reactive with the acid-containing monomer material; (3) compounds having at least two functional groups reactive with the acid-containing monomer material; and (4) polyvalent metal compounds which can form ionic cross-linkages. Preferred cross-linking agents are the di- or polyesters of unsaturated mono- or polycarboxylic acids with polyols, the bisacrylamides and the di- or triallyl amines. Especially preferred cross-linking agents are N,N'-methylenebisacrylamide, trimethylol propane triacrylate and triallyl amine. The cross-linking agent will generally comprise from about 0.001 mole percent to about 5 mole percent of the preferred materials. More preferably, the cross-linking agent will comprise from about 0.01 mole percent to about 3 mole percent of the absorbent gelling materials used herein.

The superabsorbent polymers described above are typically used in the form of discrete particles. Such superabsorbent polymers can be of any desired shape, e.g., spherical or semispherical, cubic, rod-like polyhedral, etc. Shapes having a large greatest dimension/smallest dimension ratio, like needles and flakes, are also contemplated for use herein. Agglomerates of fluid absorbent gelling material particles may also be used.

The size of the fluid absorbent gelling material particles may vary over a wide range. For reasons of industrial hygiene, average particle sizes smaller than about 30 microns are less desirable. Particles having a smallest dimension larger than about 2 mm may also cause a feeling of grittiness in the absorbent article, which is undesirable from a consumer aesthetics standpoint. Furthermore, rate of fluid absorption can be affected by particle size. Larger particles have very much reduced rates of absorption. Fluid absorbent gelling material particles preferably have a particle size of from about 30 microns to about 2 mm for substantially all of the particles. "Particle Size" as used herein means the weighted average of the smallest dimension of the individual particles.

These layers are preferably substantially free of airfelt and are thus distinct from mixed layers that may include airfelt. As used herein, "substantially free of airfelt" means less than 5%, 3%, 1%, or even 0.5% of airfelt. In a preferred case, there will be no measurable airfelt in the superabsorbent layers of the absorbent core. In the case of the first superabsorbent layer, it is preferably disposed onto the first distribution layer discontinuously. As used herein "discontinuously" or "in a discontinuous pattern" means that the superabsorbent polymers are applied onto the first distribution layer in a pattern of disconnected shaped areas. These areas of superabsorbent polymers or areas free of superabsorbent polymer may include, but are not limited to linear strips, non-linear strips, circles, rectangles, triangles, waves, mesh, and combinations thereof. The first superabsorbent layer like the second superabsorbent layer may, however, be disposed onto its respective distribution layer in a continuous pattern. As used herein "continuous pattern" or "continuously" means that the material is deposited and or secured to a superabsorbent carrier material and/or the adjacent distribution layer in an uninterrupted manner such that there is rather full coverage of the distribution layer by the superabsorbent polymer.

The absorbent core may be a heterogeneous mass comprising enrobable elements and one or more portions of foam pieces. The discrete portions of foam pieces are open-celled foam. The enrobable elements may be a web such as, for example, nonwoven, a fibrous structure, an air-laid web, a wet laid web, a high loft nonwoven, a needlepunched web, a hydroentangled web, a fiber tow, a woven web, a knitted web, a flocked web, a spunbond web, a layered spunbond/ melt blown web, a carded fiber web, a coform web of cellulose fiber and melt blown fibers, a coform web of staple fibers and melt blown fibers, and layered webs that are layered combinations thereof. The foam may be a High Internal Phase Emulsion (HIPE) foam. Exemplary enrobable elements and foams are described in greater detail below.

The open-cell foam pieces may comprise between 1% of the heterogeneous mass by volume to 99% of the heterogeneous mass by volume, such as, for example, 5% by volume, 10% by volume, 15% by volume, 20% by volume, 25% by volume, 30% by volume, 35% by volume, 40% by volume, 45% by volume, 50% by volume, 55% by volume, 60% by volume, 65% by volume, 70% by volume, 75% by volume, 80% by volume, 85% by volume, 90% by volume, or 95% by volume.

The heterogeneous mass may have void space found between the enrobeable elements, between the enrobeable elements and the enrobed elements, and between enrobed elements. The void space may contain a gas such as air. The void space may represent between 1% and 95% of the total volume for a fixed amount of volume of the heterogeneous mass, such as, for example, 5%, 10%, 15%, 20%, 25%, 30%, 35%, 40%, 45%, 50%, 55%, 60%, 65%, 70%, 75%, 80%, 85%, 90% of the total volume for a fixed amount of volume of the heterogeneous mass.

Formation means known for deforming a generally planar fibrous web into a three-dimensional structure are utilized in the present invention to modify as-made absorbent materials into absorbent materials having relatively higher permeability without a significant corresponding decrease in capillary pressure. Formation means may comprise a pair of inter-meshing rolls, typically steel rolls having inter-engaging ridges or teeth and grooves. However, it is contemplated that other means for achieving formation can be utilized, such as the deforming roller and cord arrangement disclosed in US 2005/0140057 published June 30, 2005. Therefore, all disclosure of a pair of rolls herein is considered equivalent to a roll and cord, and a claimed arrangement reciting two inter-meshing rolls is considered equivalent to an inter-meshing roll and cord where a cord functions as the ridges of a mating inter-engaging roll. In one embodiment, the pair of

intermeshing rolls of the instant invention can be considered as equivalent to a roll and an inter-meshing element, wherein the inter-meshing element can be another roll, a cord, a plurality of cords, a belt, a pliable web, or straps. Likewise, other known formation technologies, such as creping, necking/consolidation, corrugating, embossing, button break, hot pin punching, and the like are believed to be able to produce absorbent materials having some degree of relatively higher permeability without a significant corresponding decrease in capillary pressure. Formation means utilizing rolls include "ring rolling", a "SELF" or "SELF'ing" process, in which SELF stands for Structural Elastic Like Film, as "micro-SELF", and "rotary knife aperturing" (RKA); as described in US Patent No. 7,935,207 Zhao et al., granted May 3, 2011.

The distribution may be optimized dependent on the intended use of the heterogeneous mass. For example, a different distribution may be chosen for the absorption of aqueous fluids such as urine when used in a diaper or water when used in a paper towel versus for the absorption of a proteinaceous fluid such as menses. Further, the distribution may be optimized for uses such as dosing an active or to use the foam as a reinforcing element.

The absorbent core may also comprise similar optional layers. They may be webs selected from the group consisting of a fibrous structure, an airlaid web, a wet laid web, a high loft nonwoven, a needlepunched web, a hydroentangled web, a fiber tow, a woven web, a knitted web, a flocked web, a spunbond web, a layered spunbond/ melt blown web, a carded fiber web, a coform web of cellulose fiber and melt blown fibers, a coform web of staple fibers and melt blown fibers, and layered webs that are layered combinations thereof.

These optional layers of the core and of the chassis may comprise materials such as creped cellulose wadding, fluffed cellulose fibers, airlaid (airfelt), and textile fibers. The materials of the optional layers may also be fibers such as, for example, synthetic fibers, thermoplastic particulates or fibers, tricomponent fibers, and bicomponent fibers such as, for example, sheath/core fibers having the following polymer combinations: polyethylene/polypropylene, polyethylvinyl acetate/polypropylene, polyethylene/polyester, polypropylene/polyester, copolyester/polyester, and the like. The optional layers may be any combination of the materials listed above and/or a plurality of the materials listed above, alone or in combination.

The materials of the optional layers may be hydrophobic or hydrophilic depending on their placement within the chassis.

The materials of the optional layers may comprise constituent fibers comprising polymers such as polyethylene, polypropylene, polyester, and blends thereof. The fibers may be spunbound fibers. The fibers may be meltblown fibers. The fibers may comprise cellulose, rayon, cotton, or other natural materials or blends of polymer and natural materials. The fibers may also comprise a superabsorbent material such as polyacrylate or any combination of suitable materials. The fibers may be monocomponent, bicomponent, and/or biconstituent, non-round (e.g., capillary channel fibers), and may have major cross-sectional dimensions (e.g., diameter for round fibers) ranging from 0.1-500 microns. The constituent fibers of the nonwoven precursor web may also be a mixture of different fiber types, differing in such features as chemistry (e.g. polyethylene and polypropylene), components (mono- and bi-), denier (micro denier and >20 denier), shape (i.e., capillary and round) and the like. The constituent fibers may range from about 0.1 denier to about 100 denier.

The optional layers may include thermoplastic particulates or fibers. The materials, and in particular thermoplastic fibers, may be made from a variety of thermoplastic polymers including polyolefins such as polyethylene (e.g., PULPEX™) and polypropylene, polyesters, copolyesters, and copolymers of any of the foregoing.

Depending upon the desired characteristics, suitable thermoplastic materials include hydrophobic fibers that have been made hydrophilic, such as surfactant-treated or silica-treated thermoplastic fibers derived from, for example, polyolefins such as polyethylene or polypropylene, polyacrylics, polyamides, polystyrenes, and the like. The surface of the hydrophobic thermoplastic fiber may be rendered hydrophilic by treatment with a surfactant, such as a nonionic or anionic surfactant, e.g., by spraying the fiber with a surfactant, by dipping the fiber into a surfactant or by including the surfactant as part of the polymer melt in producing the thermoplastic fiber. Upon melting and resolidification, the surfactant will tend to remain at the surfaces of the thermoplastic fiber. Suitable surfactants include nonionic surfactants such as Brij 76 manufactured by ICI Americas, Inc. of Wilmington, Del., and various surfactants sold under the Pegosperse™ by Glyco Chemical, Inc. of Greenwich, Conn. Besides nonionic surfactants, anionic surfactants may also be used. These surfactants may be applied to the thermoplastic fibers at levels of, for example, from about 0.2 to about 1 g/cm$^2$ of thermoplastic fiber.

Suitable thermoplastic fibers may be made from a single polymer (monocomponent fibers), or may be made from more than one polymer (e.g., bicomponent fibers). The polymer comprising the sheath often melts at a different, typically lower, temperature than the polymer comprising the core. As a result, these bicomponent fibers provide thermal bonding due to melting of the sheath polymer, while retaining the desirable strength characteristics of the core polymer.

Suitable bicomponent fibers for use in the present invention may include sheath/core fibers having the following polymer combinations: polyethylene/polypropylene, polyethylvinyl acetate/polypropylene, polyethylene/polyester, polypropylene/polyester, copolyester/polyester, and the like. Particularly suitable bicomponent thermoplastic fibers for use herein are those having a polypropylene or polyester core, and a lower melting copolyester, polyethylvinyl acetate or polyethylene sheath (e.g., DANAKLON™, CELBOND™, or CHISSO™ bicomponent fibers). These bicomponent fibers may be concentric or eccentric. As used herein, the terms "concentric" and "eccentric" refer to whether the sheath has

a thickness that is even, or uneven, through the cross-sectional area of the bicomponent fiber. Eccentric bicomponent fibers may be desirable in providing more compressive strength at lower fiber thicknesses. Suitable bicomponent fibers for use herein may be either uncrimped (i.e., unbent) or crimped (i.e., bent). Bicomponent fibers may be crimped by typical textile means such as, for example, a stuffer box method or the gear crimp method to achieve a predominantly two-dimensional or "flat" crimp.

The optional layers may also include synthetic fibers that typically do not function as binder fibers but alter the mechanical properties of the fibrous webs. Synthetic fibers include cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. These might include, for example, polyester fibers such as polyethylene terephthalate (e.g., DACRON™, and KODEL™), high melting crimped polyester fibers (e.g., KODEL™ 431 made by Eastman Chemical Co.) hydrophilic nylon (HYDROFIL™), and the like. Suitable fibers may also hydrophilized hydrophobic fibers, such as surfactant-treated or silica-treated thermoplastic fibers derived from, for example, polyolefins such as polyethylene or polypropylene, polyacrylics, polyamides, polystyrenes, polyurethanes and the like. In the case of nonbonding thermoplastic fibers, their length may vary depending upon the particular properties desired for these fibers. Typically they have a length from about 0.3 to 7.5 cm, such as, for example from about 0.9 to about 1.5 cm. Suitable nonbonding thermoplastic fibers may have a decitex in the range of about 1.5 to about 35 decitex, such as, for example, from about 14 to about 20 decitex.

Optional Components

In varying embodiments of the invention, disposable absorbent articles of the present invention may comprise a pair of barrier cuffs that run along opposing edges of the body surface of the topsheet of the article. These cuffs run along a longitudinal axis of the chassis in a parallel configuration to one another. The pair of barrier cuffs may also be attached to the backsheet instead of the topsheet, depending on the configuration of the product. Some examples of other suitable barrier cuffs are described in U.S. Patent Nos. 4,695,278; 4,704,115; 4,795,454; 4,909,803; U.S. Patent Application Publication No. 2009/0312730.

In some forms, the first barrier cuff comprises a first cover and a first elastic member. The second barrier cuff comprises a second cover and a second elastic member. The first cover may fully enclose the first elastic member. Similarly, the second cover may fully enclose the second elastic member.

In some forms of the present invention, where the product form is an incontinence pad or sanitary napkin, such product forms may comprise wings. Wings can provide additional leakage protection for the disposable absorbent article and can help secure it pad to the underwear of the user. Any suitable wing configuration known in the art may be utilized.

All the components can be adhered together with adhesives, including hot melt adhesives, as is known in the art. The adhesive can be Findlay H2128 UN or Savare PM 17 and can be applied using a Dynafiber HTW system.

In the case of a sanitary napkin or an incontinence pad, during use, such a product can be held in place by any support or attachment suitable for such purposes. In certain forms of the present invention, the pad is placed in the user's undergarment or panty and secured thereto by the fastening adhesive. The fastening adhesive secures the pad in the crotch portion of the user's panty. A portion or all of the garment surface of the backsheet is coated with fastening adhesive. Any adhesive or glue suitable for such purposes can be used for the fastening adhesive herein, such as, for example, using pressure-sensitive adhesive. Suitable adhesives include, for example, Century A-305-IV manufactured by the Century Adhesives Corporation of Columbus, Ohio; and Instant Lock 34-2823 manufactured by the National Starch and Chemical Company of Bridgewater, N.J. Suitable adhesive fasteners are also described in U.S. Pat. No. 4,917,697. Before the absorbent article is placed in use, the pressure-sensitive adhesive is typically covered with a removable release liner in order to keep the adhesive from drying out or adhering to a surface other than the crotch portion of the panty prior to use. Suitable release liners are also described in U.S. Pat. Nos. 4,917,697 and 4,556,146. Any commercially available release liners commonly used for such purposes can be utilized herein. Non-limiting examples of suitable release liners are BL30MG-A Silox E1/0 and BL30MG-A Silox 4P/O both of which are manufactured by the Akrosil Corporation of Menasha, Wis. The pad can be used by removing the release liner and thereafter placing the absorbent article in a panty so that the adhesive contacts the panty. The adhesive maintains the absorbent article in its position within the panty during use. The release liner can also be a wrapper that can individually package the pad.

Again, although the majority of discussion herein is around sanitary napkins, it is envisioned that this invention is also useful for taped diapers, training pants which pull on, adult incontinence diapers and pants, and replaceable pads for incontinence and menses collection that might be inserted and removed after use in a disposable or durable panty or underpant.

Examples

Example 1-3

A sanitary napkin approximately 240 mm long having an hourglass shape is constructed. The napkin has a width of 80 mm. The napkin is constructed using a nonwoven web topsheet that is 25 gsm 70/30 polyethylene/polypropylene bicomponent fiber supplied by Fibertex Nonwovens (Denmark). The web of the topsheet is overbonded with an average DOE of 0.065. The web has bond sizes (0.1 inches by 0.010 inches) and spacing where there are rows with horizontal spacing of 0.12 inches between bonds and the rows are offset by 0.060 inches with no vertical spacing between the rows. These bonds are melt stabilized zones and when activated by a ring rolling process form the apertures of the aperture nonwoven as is detailed in US Patent 5,916,661. The web is treated with a surface modifying composition comprising hydrophobic particles and hydrophobic binders with each of the two components applied in sequence or as a suspension of both particles and binders. The web is wetted with any one of the surface modifying compositions detailed in Table 3. In these examples, the compositions include 0.5% by weight of WR1300 as a binder and 0.5% by weight of Aersosil R812 particles. These were dissolved in a suitable solvent which could be any one of water, ethanol, isopropyl alcohol, propylene glycol n-butyl ether, or a combination of any of these. The ratios of the binder to particles in each of these examples is reflected in Table 3 below.

Table 3. Surface Modifying Compositions

| | Target Ratio Binder: Particles | Hydrophobic Binder gsm (actual) | Particle gsm (actual) | Actual Ratio polymer: particle | Stain Area on Removed TS ($mm^2$) |
|---|---|---|---|---|---|
| Comparative Example A | N/A | N/A | N/A | N/A | 43.5 |
| Comparative Example B | 5:1 | 1.44 | 0.25 | 5.9 | 48.5 |
| Comparative Example C | 10:1 | 1.44 | 0.10 | 14.4 | 44 |
| Example 1 | 1:1 | 0.91 | 0.68 | 1.3 | 2.8 |
| Example 2 | 2:1 | 1.44 | 0.69 | 2.1 | 7 |
| Example 3 | 1:2 | 0.42 | 0.74 | 0.6 | 1.1 |

Once the solvent evaporates, the nonwoven web of the topsheet is ring rolled at a depth of engagement (DOE) of 0.065 inches to form apertures in the topsheet. The topsheet is glued to a secondary topsheet of 55 or 75 gsm spunlace (supplied by Jacob Holm, Switzerland) using a glue from Bostik Inc. of Wauwatosa, WI (Bostik H2031-C5X @ 0.005 gsi). This laminate of the topsheet and secondary topsheet is then glued using the same glue to a 160 gsm or 200 gsm airlaid core from Glatfelter of York, PA. This combination is joined to a film backsheet (14.2 gsm) from RKW (Germany). The resultant napkin is formed by heat crimp sealing around the periphery of the napkin. A panty fastening adhesive is then slot coated on the backsheet. As shown in Table 3, when the topsheet is not treated with the surface modification of the present invention as in Comparative Example A, the Stain Area of the removed topsheet is high, which is undesirable from a wearer's standpoint. Where the ratio of the binder to particles falls outside the claimed range as is the case for Comparative Examples B and C, the Stain Area of the removed topsheet is also high. Examples 1, 2, and 3 each exhibit binder to particle ratios of the present invention and the measured Stain Areas on the removed topsheets are significantly lower. Figure 3 shows a plot of these different binder to particles ratios of Examples 1, 2, and 3 versus their respective Stain Areas on the removed topsheet. These are contrasted with the values of Comparative Examples B and C, which fall outside of the claimed ranges.

Example 4-6

A sanitary napkin approximately 240 mm long having an hourglass shape is constructed. The napkin has a width of 80 mm. The napkin is constructed using a two layer topsheet. The first layer of the topsheet is a nonwoven web as detailed in Examples 1-3 with each of Examples 4-6 employing one of the three different binder to particles ratios. The second layer of the topsheet is a 25 gsm 70/30 polyethylene/polypropylene bicomponent fiber philic nonwoven supplied by Pegas (Czech Republic). The dual layer topsheet is overbonded as described in Examples 1-3. This dual

layer topsheet is fusion bonded with heat and pressure to a secondary topsheet of 55 or 75 gsm spunlace (same as used in Example 1) using the same Bostik glue (Bostik H2031-C5X at 0.005 gsi) from Example 1. This laminate of the topsheet and secondary topsheet is then glued to a 160 gsm to 200 gsm airlaid absorbent core from Glatfelter of York, PA. This combination is joined to a film backsheet (14.2 gsm) from RKW (Germany). The resultant napkin is formed by heat crimp sealing around the periphery of the napkin. A panty fastening adhesive is then slot coated on the backsheet.

## Test Methods

Various values are reported herein for the purposes of characterizing the invention. The methods for their determination are detailed below.

## Fiber Roughness (Rq)

The microscale profile fiber roughness parameter Rq (root mean square height), as described in ISO 4287, is used to characterize the roughness of an extracted profile along the top of a single nonwoven fiber. Microscale profile roughness of the fiber test samples is determined using a 3D Laser Scanning Confocal Microscope (one suitable 3D Laser Scanning Confocal Microscope is the Keyence VK-X200, commercially available from Keyence Corporation of America, Itasca, IL, USA). The microscope is interfaced with a computer running measurement and control software (suitable software programs include the Keyence VK Viewer version 2.4.1.0; the Keyence MultiFile Analyzer version 1.1.14.62; and the Keyence VK Analyzer version 3.4.0.1, all commercially available from Keyence Corporation of America, Itasca, IL, USA), and surface texture analysis software like Mountains Map, which is commercially available from Digital Surf, Besançon, France, or an equivalent. The instrument is calibrated according to the manufacturer's specifications. The 3D Laser Scanning Confocal Microscope is used in accordance with ISO 25178-2:2012 to collect topographic surface height data over a given surface area of a test fiber sample, and produce maps of surface height (i.e., z-directional or z-axis) versus displacement in the x-y plane. A profile along the length of the test fiber is extracted from the surface map and analyzed in accordance with ISO 16610 and ISO 4287, from which the profile Fiber Roughness value Rq, is calculated. The units of the reported Rq value are micrometers ($\mu$m).

Images are collected using a 150$\times$ magnification objective lens provided by the instrument manufacturer, to yield a captured image Field of View (FoV) of approximately 96 um $\times$ 72 um with an x-y resolution of approximately 94 nanometers/pixel. The microscope is programmed to collect the surface height (z-direction) image data of the FoV using a z-step size of 0.08 $\mu$m, over a height range that is sufficient to capture all relevant peaks and valleys within the given FoV. Surface height image data are acquired by following the instrument manufacturer's recommended measurement procedures, which may include using the following settings: Real Peak Detection (RPD) set to on; Zoom set to 1.0; Laser Intensity (Brightness and ND filter) set to auto gain; double scan not used; Mode set to Surface Profile; Area set to Standard (1024 $\times$ 768) pixels; Quality set to High-Accuracy.

Place the aperture nonwoven specimen on the stage beneath the objective lens such that a single fiber is visible in the FoV. Align the central axis of the fiber so that it runs parallel to the longer side of the rectangular FoV, such that it will provide a profile length for analysis of at least 90 $\mu$m. The fiber is oriented horizontally to ensure that the vertical scanning axis is as perpendicular as possible to the central axis of the fiber. Collect a surface height image (z-direction) of the fiber specimen by following the instrument manufacturer's recommended measurement procedures.

The entire image of each captured FoV is mean filtered using a 5x5 filter size prior to extracting the profile. A profile along the top of the fiber is extracted. The entire length of the profile is analyzed to determine the Rq value of the fiber in that image. In accordance with the filtration processes recommended in ISO 16610-21 and 16610-31, the following filtering procedure is performed on each profile: 1) Level using the linear least square fit of the profile; 2) a Gaussian low pass lambda s ($\lambda$s) filter with a nesting index (cut-off) of 5 $\mu$m utilizing end effect correction; 3) a robust Gaussian high pass lambda c ($\lambda$c) filter with a nesting index (cut-off) of 25 $\mu$m utilizing end effect correction. This filtering procedure produces the fiber roughness profile from which the Rq value is calculated. According to ISO 4287 Rq is defined as the root mean square deviation of the assessed profile, which corresponds to the standard deviation of the height distribution calculated on the profile (evaluation) length.

Scan and analyze the microscale profile fiber roughness of six substantially similar replicate fibers. An Rq value is recorded for each of the six replicate fiber samples to the nearest 0.001 $\mu$m. The average of the six individual Rq is reported as the Fiber Roughness value, Rq, to the nearest 0.001 $\mu$m.

## Artificial Menstrual Fluid (AMF) Preparation

The Artificial Menstrual Fluid (AMF) is composed of a mixture of defibrinated sheep blood, a phosphate buffered saline solution and a mucous component. The AMF is prepared such that it has a viscosity between 7.15 to 8.65 centistokes at 23 °C.

Viscosity on the AMF is performed using a low viscosity rotary viscometer (a suitable instrument is the Cannon LV-2020 Rotary Viscometer with UL adapter, Cannon Instrument Co., State College, PA, or equivalent). The appropriate size spindle for the viscosity range is selected, and instrument is operated and calibrated as per the manufacturer. Measurements are taken at 23 °C ± 1 C° and at 60 rpm. Results are reported to the nearest 0.01 centistokes.

Reagents needed for the AMF preparation include: defibrinated sheep blood with a packed cell volume of 38% or greater (collected under sterile conditions, available from Cleveland Scientific, Inc., Bath, OH, or equivalent), gastric mucin with a viscosity target of 3-4 centistokes when prepared as a 2% aqueous solution (crude form, available from Sterilized American Laboratories, Inc., Omaha, NE, or equivalent), 10% v/v lactic acid aqueous solution, 10% w/v potassium hydroxide aqueous solution, sodium phosphate dibasic anhydrous (reagent grade), sodium chloride (reagent grade), sodium phosphate monobasic monohydrate (reagent grade) and distilled water, each available from VWR International or an equivalent source.

The phosphate buffered saline solution consists of two individually prepared solutions (Solution A and Solution B). To prepare 1 L of Solution A, add 1.38 ± 0.005 g of sodium phosphate monobasic monohydrate and 8.50 ± 0.005 g of sodium chloride to a 1000 mL volumetric flask and add distilled water to volume. Mix thoroughly. To prepare 1 L of Solution B, add 1.42 ± 0.005 g of sodium phosphate dibasic anhydrous and 8.50 ± 0.005 g of sodium chloride to a 1000 mL volumetric flask and add distilled water to volume. Mix thoroughly. To prepare the phosphate buffered saline solution, add 450 ± 10 mL of Solution B to a 1000 mL beaker and stir at low speed on a stir plate. Insert a calibrated pH probe (accurate to 0.1) into the beaker of Solution B and add enough Solution A, while stirring, to bring the pH to 7.2 ± 0.1.

The mucous component is a mixture of the phosphate buffered saline solution, potassium hydroxide aqueous solution, gastric mucin and lactic acid aqueous solution. The amount of gastric mucin added to the mucous component directly affects the final viscosity of the prepared AMF. To determine the amount of gastric mucin needed to achieve AMF within the target viscosity range (7.15 - 8.65 centistokes at 23 °C) prepare 3 batches of AMF with varying amounts of gastric mucin in the mucous component, and then interpolate the exact amount needed from a concentration versus viscosity curve with a least squares linear fit through the three points. A successful range of gastric mucin is usually between 38 to 50 grams.

To prepare about 500 mL of the mucous component, add 460 ± 10 mL of the previously prepared phosphate buffered saline solution and 7.5 ± 0.5 mL of the 10% w/v potassium hydroxide aqueous solution to a 1000 mL heavy duty glass beaker. Place this beaker onto a stirring hot plate and while stirring, bring the temperature to 45 °C ± 5 C°. Weigh the pre-determined amount of gastric mucin (± 0.50 g) and slowly sprinkle it, without clumping, into the previously prepared liquid that has been brought to 45 °C. Cover the beaker and continue mixing. Over a period of 15 minutes bring the temperature of this mixture to above 50 °C but not to exceed 80 °C. Continue heating with gentle stirring for 2.5 hours while maintaining this temperature range. After the 2.5 hours has elapsed, remove the beaker from the hot plate and cool to below 40 °C. Next add 1.8 ± 0.2 mL of the 10% v/v lactic acid aqueous solution and mix thoroughly. Autoclave the mucous component mixture at 121°C for 15 minutes and allow 5 minutes for cool down. Remove the mixture of mucous component from the autoclave and stir until the temperature reaches 23 °C ± 1 C°.

Allow the temperature of the sheep blood and mucous component to come to 23 °C ± 1 C°. Using a 500 mL graduated cylinder, measure the volume of the entire batch of the previously prepared mucous component and add it to a 1200 mL beaker. Add an equal volume of sheep blood to the beaker and mix thoroughly. Using the viscosity method previously described, ensure the viscosity of the AMF is between 7.15 - 8.65 centistokes. If not the batch is disposed and another batch is made adjusting the mucous component as appropriate.

The qualified AMF should be refrigerated at 4 °C unless intended for immediate use. AMF may be stored in an air-tight container at 4 °C for up to 48 hours after preparation. Prior to testing, the AMF must be brought to 23 °C ± 1 C°. Any unused portion is discarded after testing is complete.

<u>Acquisition Time</u>

Acquisition Time is measured for an absorbent article loaded with Artificial Menstrual Fluid (AMF) as described herein, using a strikethrough plate and an electronic circuit interval timer. The time required for the absorbent article to acquire a dose of AMF is recorded. All measurements are performed in a laboratory maintained at 23 °C ± 2 C° and 50% ± 2% relative humidity.

Referring to Figures 4 - 6B, the strikethrough plate **9001** is constructed of Plexiglas with an overall dimension of 10.2 cm long by 10.2 cm wide by 3.2 cm tall. A longitudinal channel **9007** runs the length of the plate is 13 mm deep, 28 mm wide at the top plane of the plate, with lateral walls that slope downward at 65° to a 15 mm wide base. A central test fluid well **9009** is 26 mm long, 24 mm deep, 38 mm wide at the top plane of the plate with lateral walls that slope downward at 65° to a 15 mm wide base. At the base of the test fluid well **9009**, there is an "H" shaped test fluid reservoir **9003** open to the bottom of the plate for the fluid to be introduced onto the underlying article. The test fluid reservoir **9003** has an overall length of 25 mm, width of 15 mm, and depth of 8 mm. The longitudinal legs of the reservoir are 4

mm wide and have rounded ends with a radius **9010** of 2 mm. The legs are 3.5 mm apart. The central strut has a radius **9011** of 3 mm and houses the opposing electrodes 6 mm apart. The lateral sides of the reservoir bow outward at a radius **9012** of 14 mm bounded by the overall width **2013** of 15 mm. Two wells **9002** (80.5 mm long × 24.5 mm wide × 25 mm deep) located outboard of the lateral channel, are filled with lead shot to adjust the overall mass of the plate to provide a constraining pressure of 0.25 psi (17.6 gf/$cm^2$) to the test area. Electrodes **9004** are embedded in the plate **9001,** connecting the exterior banana jacks **9006** to the inside wall **9005** of the fluid reservoir **9003.** A circuit interval timer is plugged into the jacks **9006**, and monitors the impedance between the two electrodes **9004**, and measures the time from introduction of the AMF into reservoir **9003** until the AMF drains from the reservoir. The timer has a resolution of 0.01 sec.

Test products are removed from all packaging using care not to press down or pull on the products while handling. No attempt is made to smooth out wrinkles. The test samples are conditioned at 23 °C ± 2 C° and 50% ± 2% relative humidity for at least 2 hours prior to testing.

The required mass of the strikethrough plate must be calculated for the specific dimensions of the test article such that a confining pressure of 1.72 kPa is applied. Determine the longitudinal and lateral midpoint of the article's absorbent core. Measure and record the lateral width of the core to the nearest 0.1 cm. The required mass of the strikethrough plate is calculated as the core width multiplied by strikethrough plate length (10.2 cm) multiplied by17.6 g/$cm^2$ and recorded to the nearest 0.1 g. Add lead shot to the plate to achieve the calculated mass.

Connect the electronic circuit interval timer to the strikethrough plate **9001** and zero the timer. Place the test product onto a flat, horizontal surface with the body side facing up. Gently place the strikethrough plate **9001** onto the center of the test product ensuring that the "H" shaped reservoir **9003** is centered over the test area.

Using a mechanical pipette, accurately pipette 4.00 mL ± 0.05 mL of AMF into the test fluid reservoir **9003.** The fluid is dispensed, without splashing, along the molded lip of the bottom of the reservoir **9003** within a period of 3 seconds or less. After the fluid has been acquired, record the Acquisition Time to the nearest 0.01 second. Thoroughly clean the electrodes **9004** before each test.

In like fashion, a total of five (5) replicates samples are tested for each test product to be evaluated. Report the Acquisition Time (sec) as the arithmetic mean of the replicates to the nearest 0.01 sec.

Free Fluid Acquisition Time and Stain Perception Measurement Method

Stain perception (characterized by an observed Stain Area of the topsheet and, in some instances, Stain Chroma) is measured by the size and color intensity of a fluid stain visible on an absorbent article. Artificial menstrual fluid (AMF), prepared as described herein, is dosed onto the surface of an article, and is photographed under controlled conditions. The photographic image is then calibrated and analyzed using image analysis software to obtain measurements of the size and color intensity of the resulting visible stain. All measurements are performed at constant temperature (23 °C ± 2 C°) and relative humidity (50% ± 2%).

The absorbent article, a calibrated color standard containing 24 standard color chips (e.g., ColorChecker Passport available from X-Rite; Grand Rapids, MI, or equivalent), and a calibrated ruler (traceable to NIST, or equivalent) are laid horizontally flat on a matte black background inside a light box that provides stable uniform lighting evenly across the entire base of the light box. A suitable light box is the Sanoto MK50 (Sanoto, Guangdong, China), or equivalent, which provide an illumination of 5500 LUX at a color temperature of 5500K. A Digital Single-Lens Reflex (DSLR) camera with manual setting controls (e.g. a Nikon D40X available from Nikon Inc., Tokyo, Japan, or equivalent) is mounted directly above an opening in the top of the light box so that the entire article, color standard, and ruler are visible within the camera's field of view.

Using a standard 18% gray card (e.g., Munsell 18% Reflectance (Gray) Neutral Patch / Kodak Gray Card R-27, available from X-Rite; Grand Rapids, MI, or equivalent), the camera's white balance is custom set for the lighting conditions inside the light box. The camera's manual settings are set so that the image is properly exposed such that there is no signal clipping in any of the color channels. Suitable settings might be an aperture setting of f/11, an ISO setting of 400, and a shutter speed setting of 1/400 sec. At a focal length of 35 mm, the camera is mounted approximately 14 inches above the article. The image is properly focused, captured, and saved as a JPEG file. The resulting image must contain the entire article, color target, and calibrated ruler at a minimum resolution of 15 pixels/mm.

Absorbent article samples are conditioned at 23 °C ± 2 C° and 50% ± 2% relative humidity for 2 hours prior to testing. Place a sample article flat, with the top sheet of the product facing upward. Position the tip of a mechanical pipette about 1 cm above the center (longitudinal and lateral midpoint) of the article's absorbent core, and accurately pipette 1.00 mL ± 0.05 mL of AMF onto the surface. The fluid is dispensed without splashing, within a period of 2 seconds. As soon as the fluid makes contact with the test sample, start a timer accurate to 0.01 seconds. After the fluid has been acquired (no pool of fluid left on the surface), stop the timer and record the acquisition time to the nearest 0.01 second. Wait 2 minutes. In like fashion, a second and third dose of AMF are applied to the test sample and the acquisition times are recorded to the nearest 0.01 second. Carefully transfer the article into the light box, and place it flat onto the

matte surface beneath the camera along with the ruler and color standard. The photographic image of the AMF dosed article is captured 2 minutes after the third AMF dose.

To analyze the image it is first transferred to a computer running an image analysis software (a suitable software is MATLAB, available from The Mathworks, Inc., Natick, MA, or equivalent).

The image is color calibrated using the true tristimulus XYZ color space values provided by the manufacturer for each of the 24 color chips in the color target. If target values are given in L*a*b* they are converted to XYZ according to the standard equations. The values are identified as $X_{true1...24}$, $Y_{true1...24}$, and $Z_{true1...24}$. Using the image analysis software the mean red, green, and blue (RGB) values of each of the 24 color chips in the image are measured using a square region of interest that covers approximately 75% of the interior area of each individual color chips. These values are identified as $R_{1...24}$, $G_{1...24}$, and $B_{1...24}$. A system of 24 equations, using the $X_{true}$ and associated RGB values for each color tile, is set up according to the following example:

$$X_{true1} = \alpha_1 + \alpha_2 R_1 + \alpha_3 G_1 + \alpha_4 B_1 + \alpha_5 R_1^2 + \alpha_6 R_1 G_1 + \alpha_7 G_1^2 + \alpha_8 R_1 B_1 + \alpha_9 G_1 B_1 + \alpha_{10} B_1^2$$

$$X_{true24} = \alpha_1 + \alpha_2 R_{24} + \alpha_3 G_{24} + \alpha_4 B_{24} + \alpha_5 R_{24}^2 + \alpha_6 R_{24} G_{24} + \alpha_7 G_{24}^2 + \alpha_8 R_{24} B_{24} + \alpha_9 G_{24} B_{24} + \alpha_{10} B_{24}^2$$

A second system of 24 equations, using the $Y_{true}$ and associated RGB values for each color tile, is set up according to the following example:

$$Y_{true1} = \beta_1 + \beta_2 R_1 + \beta_3 G_1 + \beta_4 B_1 + \beta_5 R_1^2 + \beta_6 R_1 G_1 + \beta_7 G_1^2 + \beta_8 R_1 B_1 + \beta_9 G_1 B_1 + \beta_{10} B_1^2$$

$$Y_{true24} = \beta_1 + \beta_2 R_{24} + \beta_3 G_{24} + \beta_4 B_{24} + \beta_5 R_{24}^2 + \beta_6 R_{24} G_{24} + \beta_7 G_{24}^2 + \beta_8 R_{24} B_{24} + \beta_9 G_{24} B_{24} + \beta_{10} B_{24}^2$$

A third system of 24 equations, using the $Z_{true}$ and associated RGB values for each color tile, is set up according to the following example:

$$Z_{true1} = \gamma_1 + \gamma_2 R_1 + \gamma_3 G_1 + \gamma_4 B_1 + \gamma_5 R_1^2 + \gamma_6 R_1 G_1 + \gamma_7 G_1^2 + \gamma_8 R_1 B_1 + \gamma_9 G_1 B_1 + \gamma_{10} B_1^2$$

$$Z_{true24} = \gamma_1 + \gamma_2 R_{24} + \gamma_3 G_{24} + \gamma_4 B_{24} + \gamma_5 R_{24}^2 + \gamma_6 R_{24} G_{24} + \gamma_7 G_{24}^2 + \gamma_8 R_{24} B_{24} + \gamma_9 G_{24} B_{24} + \gamma_{10} B_{24}^2$$

Using the 24 $X_{true}$ equations, each of the ten $\alpha$ coefficients are solved for using the standard equation y=Ax, where y are the $X_{true}$, $Y_{true}$, and $Z_{true}$ vectors, A is the list of the measured RGB intensities, and x is a vector of the unknown alpha ($\alpha$), beta ($\beta$), or gamma ($\gamma$) coefficients to be estimated.

For example, to solve for the $\alpha$'s in the transform that converts the RGB colors into colorimetric X tristimulus value, the arrays are as follows:

$$\hat{x} = \begin{bmatrix} \alpha_1 \\ \vdots \\ \alpha_{10} \end{bmatrix}$$

$$A = \begin{bmatrix} 1 & R_1 & G_1 & B_1 & R_1^2 & \cdots & B_1^2 \\ \vdots & \vdots & \vdots & \vdots & \vdots & \ddots & \vdots \\ 1 & R_{24} & G_{24} & B_{24} & R_{24}^2 & \cdots & B_{24}^2 \end{bmatrix}$$

$$y = \begin{bmatrix} X_{true1} \\ \vdots \\ X_{true24} \end{bmatrix}$$

The solution of the normal equations for x provides the least squares solution for the ten $\alpha$ coefficients according to the following equation:

$$\hat{x} = (A^T A)^{-1} A^T y$$

This procedure is repeated using the 24 $Y_{true}$ equations to solve for the ten $\beta$ coefficients, and the 24 $Z_{true}$ equations to solve for the ten $\gamma$ coefficients.

These coefficients are then plugged back into the original equations to provide three transform equations one

each for X, Y, and Z, by which the RGB values for each individual pixel in the image are transformed into calibrated XYZ values. For example, the RGB transform equation for X using the 10 $\alpha$ coefficients is as follows:

$$X = \alpha_1 + \alpha_2 R + \alpha_3 G + \alpha_4 B + \alpha_5 R^2 + \alpha_6 RG + \alpha_7 G^2 + \alpha_8 RB + \alpha_9 GB + \alpha_{10} B^2$$

The XYZ values are then converted into CIE 1976 L*a*b* values as defined in CIE 15:2004 section 8.2.1.1 using D65 reference white.

The image resolution is calibrated using the calibrated distance scale in the image to determine the number of pixels per millimeter.

Separate images are generated for each of the individual L*, a*, and b* channels. The Chroma image is calculated using the following formula:

$$Chroma = \sqrt{(a^*)^2 + (b^*)^2}$$

Where a* and b* are the individual colorimetric images. The Chroma image is analyzed by manually drawing the region of interest (ROI) boundary around the visibly discernable perimeter of the entire AMF stain. The area of the ROI is calculated and reported as the Overall Stain Area to the nearest 0.1 $mm^2$ and the mean Chroma value within the ROI is calculated and recorded to the nearest 0.1 units.

The same ROI is analyzed for the a* image alone, and the mean a* value within the ROI is calculated and recorded to the nearest 0.1 units.

A minimum bounding rectangle is drawn around the ROI. This is the smallest rectangle that can be drawn within which all of the points of the ROI lie. The edges of the rectangle are parallel and perpendicular to the longitudinal and lateral axis of the absorbent article, such that the ROI height (H) is defined as the height of the bounding rectangle along the longitudinal axis of the article, and the ROI width (W) is defined as the width of the bounding rectangle along the lateral axis of the article. Both H and W are recorded to the nearest 0.1 mm.

The Chroma image is threshold at a value of 24 to generate a binary image. In the binary image, the regions with a Chroma value greater than 24 appear as black, with a Gray Level (GL) value of 255, and remaining area as white, with a GL value of 0. Using the image analysis program, analyze each of the discrete black regions. Measure and record the areas of the individual discrete black regions to the nearest 0.1 $mm^2$, including any regions along the edges of the image. Sum all of the recorded areas to obtain the total area and report this value as the Interfacial Fluid Area to the nearest 0.1 $mm^2$.

The Topsheet Stain Area (i.e., of the removed topsheet) is measured by analyzing the AMF stained topsheet layer of the absorbent article sample. The article is set aside for approximately 30 min. after the AMF loading procedure, allowing the fluid on the article surface to fully dry. The entire topsheet layer of the article is carefully separated from the underlying layers and placed flat on a white background. The corners and edges of the topsheet are taped down such that its original longitudinal and lateral extension is maintained. A photographic image of the topsheet layer is collected, calibrated, and Chroma image generated according to the previously described procedures. The Chroma image is thresheld at a value which separates the regions containing dried AMF stain from the unstained regions on the topsheet to generate a binary image. In the binary image the regions with a Chroma value greater than the threshold value appear as black, with a Gray Level (GL) value of 255, and remaining area as white, with a GL value of 0. Using the image analysis program, analyze each of the discrete black regions. Measure and record the areas of the individual discrete black regions to the nearest 0.1 $mm^2$, including any regions along the edges of the image. Sum all of the recorded areas to obtain the total area and report this value as the Topsheet Stain Area (i.e., of the removed topsheet) to the nearest 0.1 $mm^2$.

This entire procedure is repeated on five substantially similar replicate articles. The reported value is the average of the five individual recorded measurements for Free Fluid Acquisition Time (first, second and third) to the nearest 0.01 second, Overall Stain Area to the nearest 0.1 $mm^2$, mean Chroma and a* to the nearest 0.1 units, H and W to the nearest 0.1 mm, Interfacial Fluid Area to the nearest 0.01 $mm^2$, and Topsheet Stain Area (i.e., removed topsheet) to the nearest 0.1 $mm^2$.

% Effective Area, Aperture Dimension and Inter-Aperture Distance Measurement Method

Effective aperture dimensions, % effective area and inter-aperture distance measurements are obtained from aperture specimen images acquired using a flatbed scanner. The scanner is capable of scanning in reflectance mode at a resolution of 6400 dpi and 8 bit grayscale (a suitable scanner is an Epson Perfection V750 Pro from Epson America Inc., Long Beach CA or equivalent). The scanner is interfaced with a computer running an image analysis program (a

suitable program is ImageJ v. 1.47 or equivalent, National Institute of Health, USA). The specimen images are distance calibrated against an acquired image of a ruler certified by NIST. The aperture specimen is backed with a black glass tile (P/N 11-0050-30, available from HunterLab, Reston, VA) prior to acquiring the image. The resulting image is then thresheld, separating open aperture regions from specimen material regions, and analyzed using the image analysis program. All testing is performed in a conditioned room maintained at about 23 ± 2 °C and about 50 ± 2 % relative humidity.

Sample Preparation

To obtain a specimen, tape the absorbent article to a rigid flat surface in a planar configuration. Any leg elastics may be cut to facilitate laying the article flat. Identify and mark on the apertured layer the outer boundary of the region lying above the absorbent core of the article. Using a razor blade excise the apertured layer specimen from the underlying layers of the article around the outer perimeter of the article. The apertured layer specimen is carefully removed such that its longitudinal and lateral extension is maintained to avoid distortion of the apertures. A cryogenic spray (such as Cyto-Freeze, Control Company, Houston TX) can be used to remove the specimen from the underlying layers if necessary. Five replicates obtained from five substantially similar articles are prepared for analysis. An apertured substrate raw material is prepared for testing by extending or activating it under the same process conditions, and to the same extent, as it would be for use on the absorbent article. Condition the samples at about 23 °C ± 2 C° and about 50% ± 2% relative humidity for 2 hours prior to testing.

Image acquisition

Place the ruler on the scanner bed, oriented parallel to the sides of the scanner glass, and close the lid. Acquire a calibration image of the ruler in reflectance mode at a resolution of 6400 dpi (approximately 252 pixels per mm) and 8 bit grayscale. Save the calibration image as an uncompressed TIFF format file. Lift the lid and remove the ruler. After obtaining the calibration image, all specimens are scanned under the same scanning conditions and measured based on the same calibration file. Next, place the apertured specimen onto the center of the scanner bed, lying flat, with the outward facing surface of the specimen facing the scanner's glass surface. The corners and edges of the specimen are secured such that its original longitudinal and lateral extension, as on the article prior to removal, is restored. Orient the specimen so that the machine direction (MD) and cross direction (CD) of the apertured specimen layer are aligned parallel with and perpendicular to the sides of the scanner's glass surface, so that the resulting specimen image will have the MD vertically running from top to bottom. Place the black glass tile on top of the specimen, close the lid and acquire a scanned image of the entire specimen. Save the specimen image as an uncompressed TIFF format file. Scan and save the remaining four replicates in like fashion. Prior to analysis, crop all specimen images to the largest rectangular field of view contained within the apertured region which had been located above the absorbent core of the article, and resave the files.

% Effective Aperture Area Calculation

Open the calibration image file in the image analysis program and perform a linear distance calibration using the imaged ruler. This distance calibration scale will be applied to all subsequent specimen images prior to analysis. Open a specimen image in the image analysis program and set the distance scale. View the 8 bit histogram (0 to 255, with one bin per GL) and identify the gray level (GL) value for the minimum population located between the dark pixel peak of the aperture holes and the lighter pixel peak of the specimen material. Threshold the image at the minimum gray level value to generate a binary image. In the binary image the apertures appear as black, with a GL value of 255, and specimen as white, with a GL value of 0.

Using the image analysis program, analyze each of the discrete aperture regions. Measure and record all of the individual aperture areas to the nearest 0.01 $mm^2$, including partial apertures along the edges of the image. Discard any apertures with an area less than 0.3 $mm^2$ as "non-effective". Sum the remaining "effective" aperture areas (including whole and partial apertures), divide by the total area included in the image and multiply by 100. Record this value as the % effective area to the nearest 0.01%.

In like fashion, analyze the remaining four specimen images. Calculate and report the average % effective area values to the nearest 0.01% for the five replicates.

Effective Aperture Dimension Measurements

Open the calibration image (containing the ruler) file in the image analysis program. Resize the resolution of the original image from 6400 dpi to 640 dpi (approximately 25.2 pixels per mm) using a bicubic interpolation. Perform a linear distance calibration using the imaged ruler. This distance calibration scale will be applied to all subsequent specimen

images prior to analysis. Open a specimen image in the image analysis program. Resize the resolution of the original image from 6400 dpi to 640 dpi (approximately 25.2 pixels per mm) using a bicubic interpolation. Set the distance scale. View the 8 bit histogram (0 to 255, with one bin per GL) and identify the gray level (GL) value for the minimum population located between the dark pixel peak of the aperture holes and the lighter pixel peak of the specimen material. Threshold the image at the minimum gray level value to generate a binary image. In the binary image the apertures appear as black, with a GL value of 255, and specimen as white, with a GL value of 0. Next, two morphological operations are performed on the binary image. First, a closing (a dilation operation followed by an erosion operation, iterations=1, pixel count=1), which removes stray fibers within an aperture hole. Second, an opening (an erosion operation followed by a dilation operation, iterations=1, pixel count=1), which removes isolated black pixels. Pad the edges of the image during the erosion step to ensure that black boundary pixels are maintained during the operation. Lastly, fill any remaining voids enclosed within the black aperture regions.

Using the image analysis program, analyze each of the discrete aperture regions. During the analysis exclude measurements of partial apertures along the edges of the image, so that only whole apertures are measured. Measure and record all of the individual aperture areas, perimeters, feret diameters (length of the apertures) along with its corresponding angle of orientation in degrees from 0 to 180, and minimum feret diameters (width of the apertures). Record the measurements for each of the individual aperture areas to the nearest 0.01 $mm^2$, the perimeters and feret diameters (length and width), to the nearest 0.01 mm, and angles to the nearest 0.01 degree. Discard any apertures with an area less than 0.3 $mm^2$ as "non-effective". Record the number of remaining "effective" apertures, divide by the area of the image and record as the Aperture Density value to the nearest 0.1 apertures per $cm^2$. The angle of orientation for an aperture aligned with the MD (vertical in the image) will have an angle of 90 degrees. Apertures with a positive slope, increasing from left to right, will have an angle between zero and 90 degrees. Apertures with a negative slope, decreasing from left to right, will have an angle between 90 and 180 degrees. Using the individual aperture angles calculate an Absolute Aperture Angle by subtracting 90 degrees from the original angle of orientation and taking its absolute value. In addition to these measurements, calculate an Aspect Ratio value for each individual aperture by dividing the aperture length by its width. Repeat this analysis for each of the remaining four replicate images. Calculate and report the statistical mean and standard deviation for each of the effective aperture dimensions (area, perimeter, length, width, and angle), the Absolute Aperture Angle and the Aspect Ratio measurements using all of the aperture values recorded from the replicates. Calculate and report the % relative standard deviation (RSD) for each of the effective aperture dimensions, the Absolute Aperture Angle and the Aspect Ratio measurements by dividing the standard deviation by the mean and multiplying by 100.

Inter-Aperture Distance Measurements

The mean, standard deviation, median, and maximum distance between the apertures can be measured by further analyzing the binary image that was analyzed for the aperture dimension measurements. First, obtain a duplicate copy of the resized binary image following the morphological operations, and using the image analysis program, perform a Voronoi operation. This generates an image of cells bounded by lines of pixels having equal distance to the borders of the two nearest pattern apertures, where the pixel values are outputs from a Euclidian distance map (EDM) of the binary image. An EDM is generated when each inter-aperture pixel in the binary image is replaced with a value equal to that pixel's distance from the nearest pattern aperture. Next, remove the background zeros to enable statistical analysis of the distance values. This is accomplished by using the image calculator to divide the Voronoi cell image by itself to generate a 32-bit floating point image where all of the cell lines have a value of one, and the remaining parts of the image are identified as Not a Number (NaN). Lastly, using the image calculator, multiply this image by the original Voronoi cell image to generate a 32-bit floating point image where the distance values along the cell lines remain, and all of the zero values have been replaced with NaN. Next, convert the pixel distance values into actual inter-aperture distances by multiplying the values in the image by the pixel resolution of the image (approximately 0.04 mm per pixel), and then multiply the image again by 2 since the values represent the midpoint distance between apertures. Measure and record the mean, standard deviation, median and maximum inter-aperture distances for the image to the nearest 0.01 mm. Repeat this procedure for all replicate images. Calculate the % relative standard deviation (RSD) for the inter-aperture distance by dividing the standard deviation by the mean and multiplying by 100.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A disposable absorbent article comprising:

   a. an apertured nonwoven topsheet having a body surface and an opposing garment surface, wherein said body surface has an effective aperture area of greater than 1.8 $mm^2$ and less than 3.5 $mm^2$;
   b. a backsheet;
   c. an absorbent core disposed between said topsheet and said backsheet;
   d. a surface modifying composition disposed on said body surface of said topsheet, said composition comprising hydrophobic particles and a hydrophobic binder in a ratio of binder to particles of 4:1 to 1:4, wherein the ratio is measured on a weight basis, and wherein, post insult, said article has a Topsheet Stain Area is less than 30 $mm^2$ as determined by the method for measuring the Topsheet Stain Area as defined in the test methods section herein.

2. The disposable absorbent article of claim 1 wherein the ratio of binder to particles is from 2:1 to 1:2.

3. The disposable absorbent article of claim 2 wherein the ratio of binder to particles is 1:1.

4. The disposable absorbent article of any of claims 1 to 3, wherein said apertured nonwoven topsheet comprises a constituent material that is hydrophobic.

5. The disposable absorbent article of any of claims 1 to 3, wherein said apertured nonwoven topsheet comprises a constituent material that is hydrophilic.

6. The disposable absorbent article of any of claims 1 to 5, wherein said topsheet exhibits a Fiber Roughness of 1 $\mu$m to 3.5 $\mu$m.

7. The disposable absorbent article of any of claims 1 to 6, wherein said surface modifying composition is coated in an amount of 0.1 gsm to 3 gsm, on one or more of a front, central, and rear portion of said body surface of said topsheet.

8. The disposable absorbent article of any of claims 1 to 7, wherein the hydrophobic particles are selected from the group consisting of hydrophobically modified silicas, modified polyacrylates, polymethacrylates, acrylate-vinylacetate copolymers, styrene acrylic copolymers, carboxylated styrene butadiene copolymers, and combinations thereof.

9. The disposable absorbent article of any of claims 1 to 8, wherein the hydrophobic binder is selected from the group consisting of hydrophobic silicones, hydrophobic amino silicones, dimethylsilicones, hydrophobic triglycerides, soy waxes, polyols, and combinations thereof.

10. The disposable absorbent article of any of claims 1 to 9, wherein said topsheet exhibits an average % effective area of less than or equal to 20%.

11. The disposable absorbent article of any of claims 1 to 10, wherein the article is selected from the group consisting of a sanitary napkin, a pantiliner, a diaper, a pant, and an incontinence pad.

**Patentansprüche**

1. Einweg-Absorptionsartikel, umfassend:

   a. eine mit Öffnungen versehene Vliesoberschicht, die eine Körperoberfläche und eine gegenüberliegende Kleidungsstückoberfläche aufweist, wobei die Körperoberfläche einen wirksamen Öffnungsbereich von mehr als 1,8 $mm^2$ und weniger als 3,5 $mm^2$ aufweist;
   b. eine Unterschicht;
   c. einen Absorptionskern, der zwischen der Oberschicht und der Unterschicht angeordnet ist;
   d. eine oberflächenmodifizierende Zusammensetzung, die auf der Körperoberfläche der Oberschicht angeordnet ist, die Zusammensetzung umfassend hydrophobe Teilchen und ein hydrophobes Bindemittel in einem Verhältnis von Bindemittel zu Teilchen von 4 : 1 bis 1 : 4, wobei das Verhältnis auf einer Gewichtsbasis gemessen wird, und wobei der Artikel, nach Insult, einen Oberschichtfleckenbereich aufweist, der weniger als 30 $mm^2$

beträgt, wie durch das Verfahren zum Messen des Oberschichtfleckenbereichs bestimmt, wie in dem Prüfverfahrenabsatz hierin definiert.

2. Einweg-Absorptionsartikel nach Anspruch 1, wobei das Verhältnis von Bindemittel zu Teilchen von 2 : 1 bis 1 : 2 ist.

3. Einweg-Absorptionsartikel nach Anspruch 2, wobei das Verhältnis von Bindemittel zu Teilchen 1 : 1 ist.

4. Einweg-Absorptionsartikel nach einem der Ansprüche 1 bis 3, wobei die mit Öffnungen versehene Vliesoberschicht ein Bestandteilmaterial umfasst, das hydrophob ist.

5. Einweg-Absorptionsartikel nach einem der Ansprüche 1 bis 3, wobei die mit Öffnungen versehene Vliesoberschicht ein Bestandteilmaterial umfasst, das hydrophil ist.

6. Einweg-Absorptionsartikel nach einem der Ansprüche 1 bis 5, wobei die Oberschicht eine Faserrauheit von 1 $\mu$m bis 3,5 $\mu$m aufweist.

7. Einweg-Absorptionsartikel nach einem der Ansprüche 1 bis 6, wobei die oberflächenmodifizierende Zusammensetzung in einer Menge von 0,1 Gramm pro Quadratmeter bis 3 Gramm pro Quadratmeter auf einem oder mehreren von einem vorderen, zentralen und hinteren Abschnitt der Körperoberfläche der Oberschicht beschichtet ist.

8. Einweg-Absorptionsartikel nach einem der Ansprüche 1 bis 7, wobei die hydrophoben Teilchen aus der Gruppe ausgewählt sind, bestehend aus hydrophob modifizierten Kieselsäuren, modifizierten Polyacrylaten, Polymethacrylaten, Acrylat-Vinylacetatcopolymeren, Styrolacrylcopolymeren, carboxylierten Styrolbutadiencopolymeren und Kombinationen davon.

9. Einweg-Absorptionsartikel nach einem der Ansprüche 1 bis 8, wobei das hydrophobe Bindemittel aus der Gruppe ausgewählt ist, bestehend aus hydrophoben Silikonen, hydrophoben Aminosilikonen, Dimethylsilikonen, hydrophoben Triglyceriden, Sojawachsen, Polyolen und Kombinationen davon.

10. Einweg-Absorptionsartikel nach einem der Ansprüche 1 bis 9, wobei die Oberschicht einen durchschnittlichen wirksamen %-Bereich von weniger als oder gleich 20 % vorweist.

11. Einweg-Absorptionsartikel nach einem der Ansprüche 1 bis 10, wobei der Artikel aus der Gruppe ausgewählt ist, bestehend aus einer Damenbinde, einer Slipeinlage, einer Windel, einem Höschen und einer Inkontinenzeinlage.

## Revendications

1. Article absorbant jetable comprenant :

a. une feuille de dessus en nontissé perforée ayant une surface corporelle et une surface de vêtement opposée, dans laquelle ladite surface corporelle a une aire efficace des perforations supérieure à 1,8 $mm^2$ et inférieure à 3,5 $mm^2$ ;
b. une feuille de fond ;
c. une âme absorbante disposée entre ladite feuille de dessus et ladite feuille de fond ;
d. une composition de modification de surface disposée sur ladite surface corporelle de ladite feuille de dessus, ladite composition comprenant des particules hydrophobes et un liant hydrophobe en un rapport du liant aux particules de 4:1 à 1:4, dans laquelle le rapport est mesuré sur une base de poids, et, après un afflux, ledit article ayant une aire de tache de la feuille de dessus est inférieure à 30 $mm^2$ telle que déterminée par le procédé destiné à mesurer la zone de tache de la feuille de dessus tel que défini dans la section des procédés de test ici.

2. Article absorbant jetable selon la revendication 1 dans lequel le rapport du liant aux particules va de 2:1 à 1:2.

3. Article absorbant jetable selon la revendication 2 dans lequel le rapport du liant aux particules est de 1:1.

4. Article absorbant jetable selon l'une quelconque des revendications 1 à 3, dans lequel ladite feuille de dessus en nontissé perforée comprend un matériau constitutif qui est hydrophobe.

5. Article absorbant jetable selon l'une quelconque des revendications 1 à 3, dans lequel ladite feuille de dessus en nontissé perforée comprend un matériau constitutif qui est hydrophile.

6. Article absorbant jetable selon l'une quelconque des revendications 1 à 5, dans lequel ladite feuille de dessus présente une rugosité des fibres de 1 μm à 3,5 μm.

7. Article absorbant jetable selon l'une quelconque des revendications 1 à 6, dans lequel ladite composition de modification de surface est revêtue en une quantité de 0,1 g/m2 à 3 g/m2, sur une ou plusieurs parties parmi une partie avant, une partie centrale, et une partie arrière de ladite surface corporelle de ladite feuille de dessus.

8. Article absorbant jetable selon l'une quelconque des revendications 1 à 7, dans lequel les particules hydrophobes sont choisies dans le groupe constitué de silices modifiées pour être rendues hydrophobes, polyacrylates modifiés, polyméthacrylates, copolymères d'acrylate-acétate de vinyle, copolymères de styrène-acrylique, copolymères de styrène-butadiène carboxylés, et des combinaisons de ceux-ci.

9. Article absorbant jetable selon l'une quelconque des revendications 1 à 8, dans lequel le liant hydrophobe est choisi dans le groupe constitué de silicones hydrophobes, aminosilicones hydrophobes, diméthylsilicones, triglycérides hydrophobes, cires de soja, polyols, et des combinaisons de ceux-ci.

10. Article absorbant jetable selon l'une quelconque des revendications 1 à 9, dans lequel ladite feuille de dessus présente un % moyen d'aire efficace inférieur ou égal à 20 %.

11. Article absorbant jetable selon l'une quelconque des revendications 1 à 10, dans lequel l'article est choisi dans le groupe constitué de serviette hygiénique, protège-slip, couche, culotte, et serviette d'incontinence.

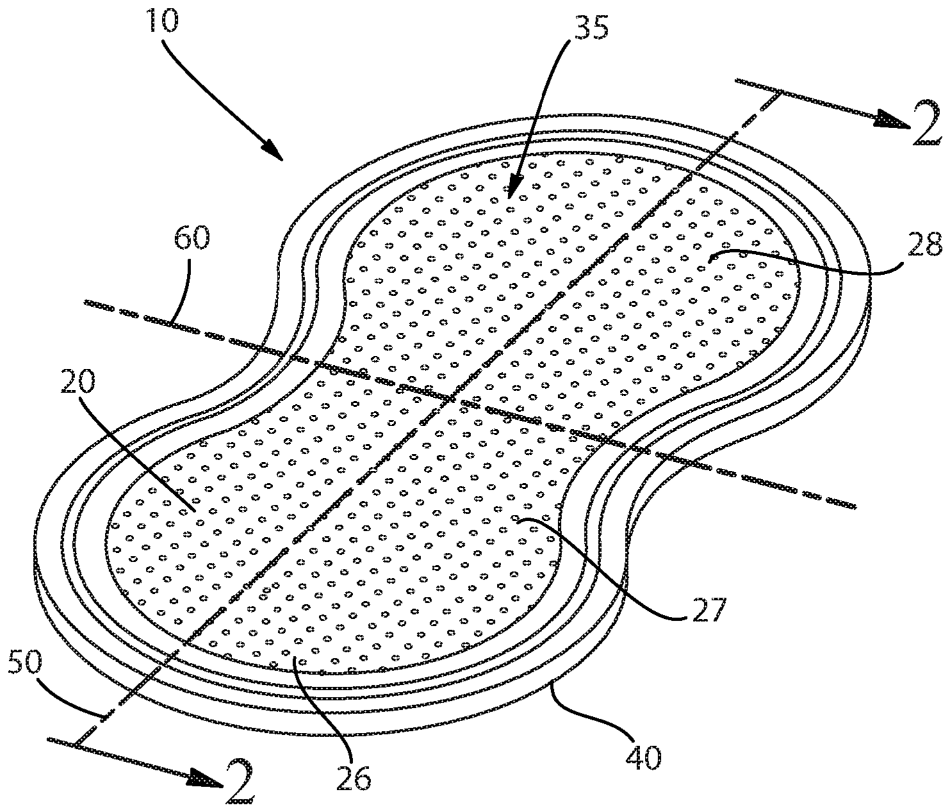


Fig. 1

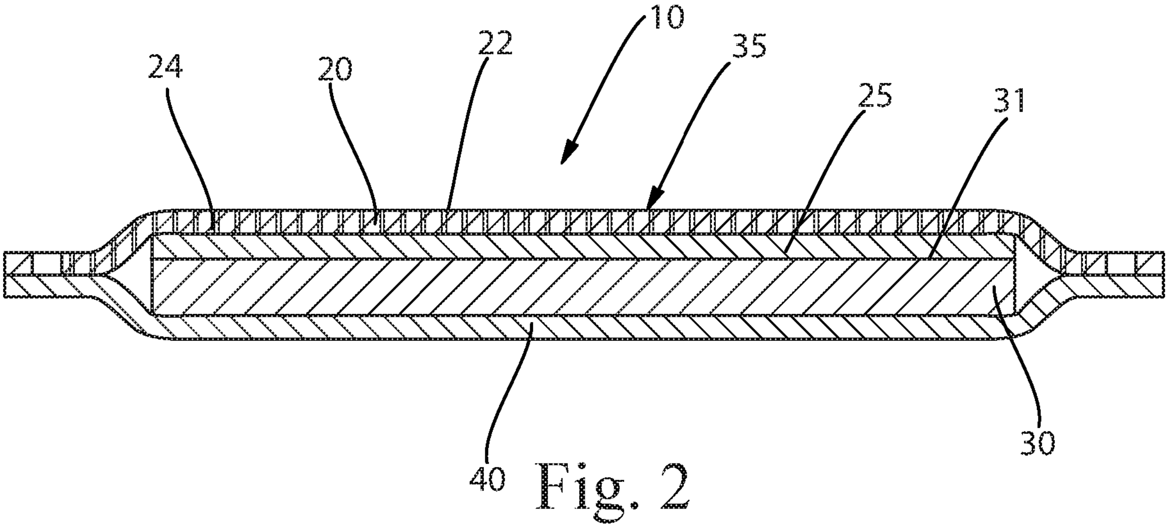


Fig. 2

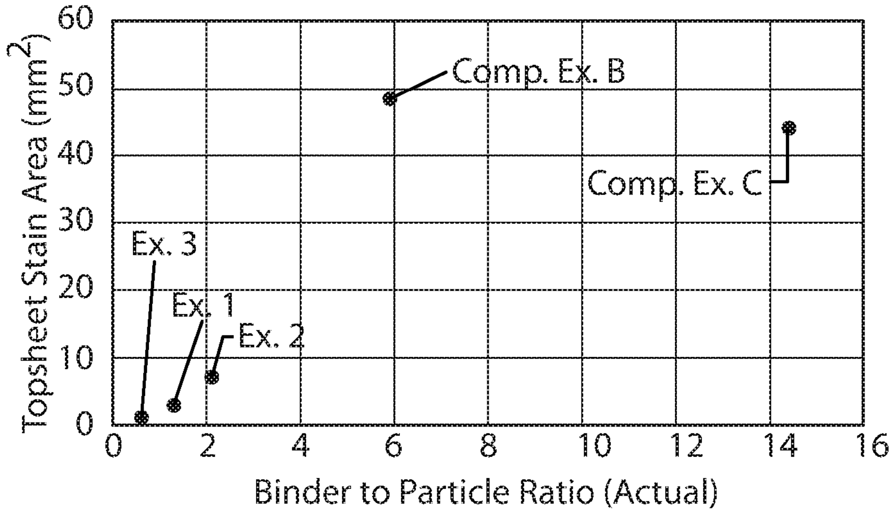
Topsheet Stain Area ($mm^2$)
60
50
40
30
20
10
0
0
2
4
6
8
10
12
14
16
Binder to Particle Ratio (Actual)
Comp. Ex. B
Comp. Ex. C
Ex. 3
Ex. 1
Ex. 2

Fig. 3

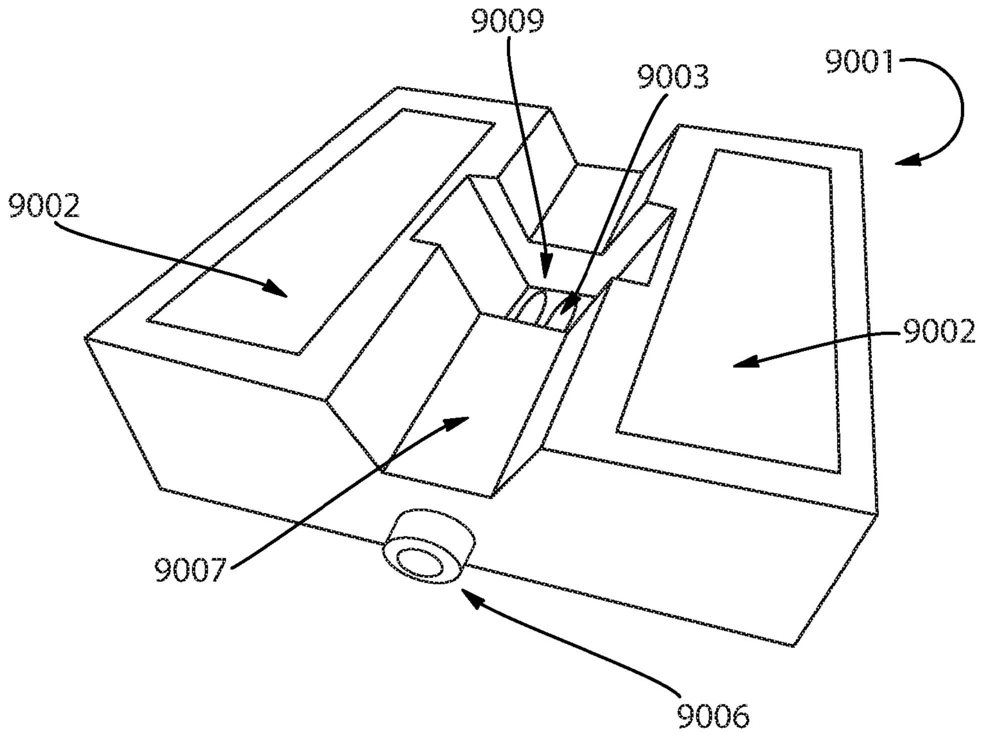


Fig. 4

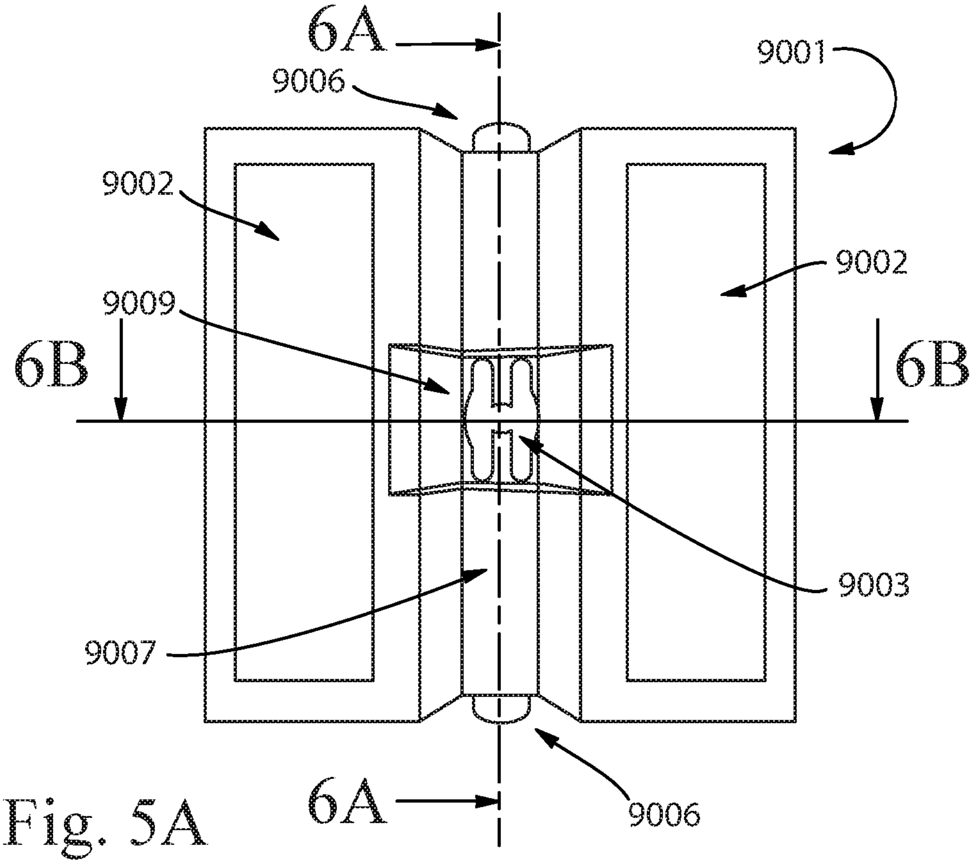
6A
9006
9001
9002
9002
9009
6B
6B
9003
9007
9006
6A

Fig. 5A

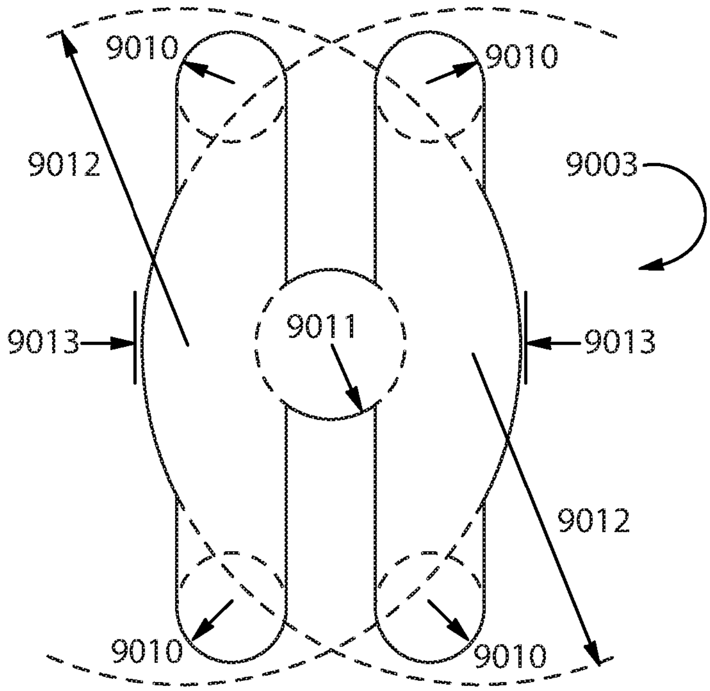
9010
9010
9012
9003
9013
9011
9013
9012
9010
9010

Fig. 5B

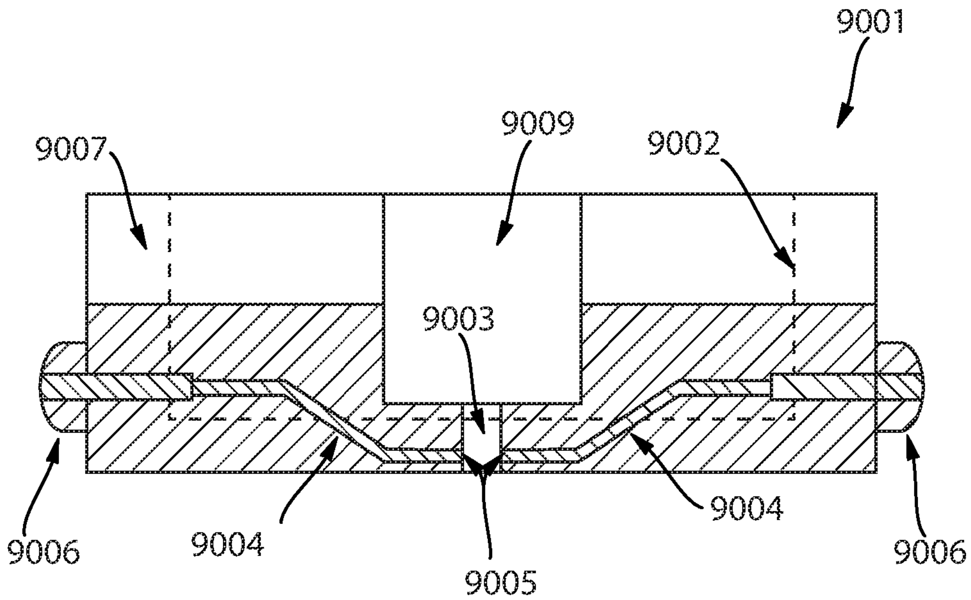
9001
9007
9009
9002
9003
9006
9004
9005
9004
9006

Fig. 6A

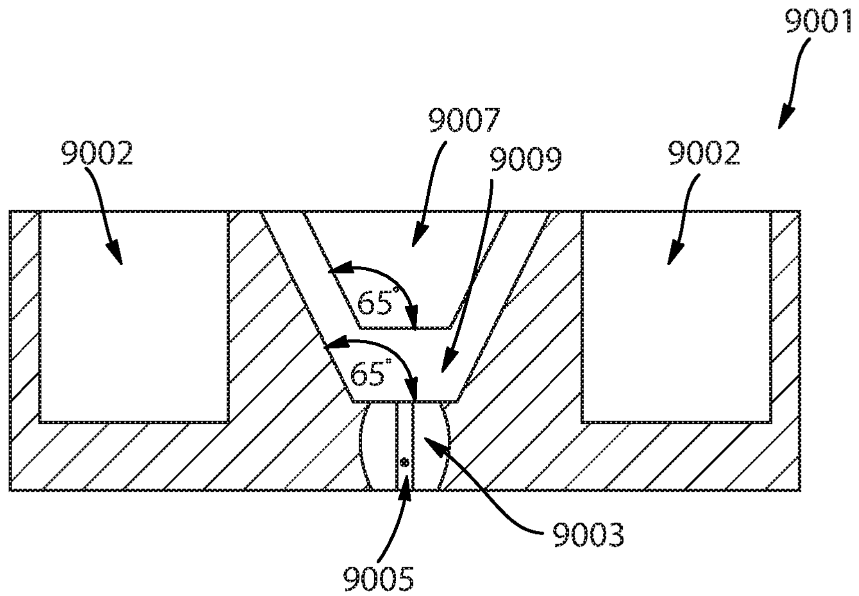
9001
9007
9002
9009
9002
65°
65°
9003
9005

Fig. 6B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2012089110 A
- WO 2015066261 A
- US 4988344 A
- US 4988345 A, Reising
- US 5916661 A, Benson
- US 7785690 B
- US 7838099 B
- US 5792404 A
- US 5665452 A
- US 8728049 B
- US 7553532 B
- US 7172801 B
- US 8440286 B
- US 7648752 B
- US 7410683 B
- US 7655176 B
- US 7402723 B
- US 8614365 B
- US 8704036 B
- US 6025535 A
- US 45399717, Jill M. Orr
- US 5628097 A, Benson
- US 5518801 A, Chappell
- US 20050140057 A
- US 7935207 B, Zhao
- US 20160129661 A
- US 20160129662 A, Aurora
- US 20090220443 A1
- US 20130344012 A1
- US 20140357714 A1
- US 5885265 A, Osborn
- US 4342314 A, Radel
- US 4463045 A, Ahr
- US 4695422 A
- US 4839216 A
- US 4591523 A
- US 3989867 A
- US 3156242 A
- WO 9724097 A
- US 6462251 B
- US 3881489 A
- US 4341216 A
- US 4713068 A
- US 4818600 A
- EP 203821 A
- EP 710471 A
- EP 710472 A
- EP 793952 A
- US 6623464 B, Bewick-Sonntag
- US 6664439 B, Arndt
- US 6436508 B, Ciammaichella
- US 20140163500
- US 20140163506 A
- US 20140163511 A
- US 5599335 A, Goldman
- EP 1447066 A, Busam
- WO 9511652 A, Tanzer
- US 20080312622 A1, Hundorf
- WO 2012052172 A, Van Malderen
- US 4610678 A, Weisman
- US 4673402 A, Weisman
- US 4888231 A, Angstadt
- US 4834735 A, Alemany
- US 5234423 A, Alemany
- US 5147345 A
- US 4695278 A
- US 4704115 A
- US 4795454 A
- US 4909803 A
- US 20090312730
- US 4917697 A
- US 4556146 A